(19) Europäisches Patentamt
European Patent Office
Office européen des brevets

(11) EP 1 904 048 B1

(12) **EUROPEAN PATENT SPECIFICATION**

(45) Date of publication and mention of the grant of the patent:
**23.11.2011 Bulletin 2011/47**

(21) Application number: **06777795.3**

(22) Date of filing: **14.07.2006**

(51) Int Cl.:
***A61K 31/192*** (2006.01) ***A61K 31/195*** (2006.01)
***A61K 31/196*** (2006.01) ***A61P 29/00*** (2006.01)
***A61P 37/00*** (2006.01) ***A61K 31/452*** (2006.01)
***A61K 31/404*** (2006.01)

(86) International application number:
**PCT/EP2006/064288**

(87) International publication number:
**WO 2007/009959 (25.01.2007 Gazette 2007/04)**

(54) **GLEPP-1 INHIBITORS IN THE TREATMENT OF AUTOIMMUNE AND/OR INFLAMMATORY DISORDERS**

GLEPP-1-HEMMER BEI DER BEHANDLUNG VON AUTOIMMUN- UND/ODER ENTZÜNDUNGSKRANKHEITEN

INHIBITEURS GLEPP-1 DANS LE TRAITEMENT DE TROUBLES AUTOIMMUNES ET/OU INFLAMMATOIRES

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **15.07.2005 EP 05106547**
**08.08.2005 US 706365 P**

(43) Date of publication of application:
**02.04.2008 Bulletin 2008/14**

(73) Proprietor: **Merck Serono SA**
**1267 Coinsins, Vaud (CH)**

(72) Inventors:
- **BOMBRUN, Agnes**
**CH-1292 Chambesy (CH)**
- **HOOFT VAN HUIJSDUIJNEN, Rob**
**CH-1233 Bernex (CH)**
- **JORAND-LEBRUN, Catherine**
**F-74270 Contamine-Sarzin (FR)**
- **VITTE, Pierre-Alain**
**F-74380 Cranves-Sales (FR)**
- **GERBER, Patrick**
**CH-1163 Etoy (CH)**

(74) Representative: **Merck Serono SA - Geneva Intellectual Property**
**9, chemin des Mines**
**1202 Geneva (CH)**

(56) References cited:
**WO-A-96/33181** **WO-A-03/032999**
**WO-A-03/059871** **WO-A-2004/080377**
**WO-A-2005/007616** **WO-A-2005/097773**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 904 048 B1

## Description

Field of the invention

The present invention is related to the use of Glepp-1 inhibitors for the manufacture of a medicament for the treatment of autoimmune and/or inflammatory disorders.

Background of the invention

Protein-tyrosine phosphatases (PTPs) play an important role in the regulation of phosphorylation of proteins and represent the counterparts of kinases. Among classical PTPs, there are two types : (i) non-receptor or intracellular PTPs and (ii) receptor-like PTPs. Most intracellular PTPs contain one catalytic domain only, whereas most receptor-like enzymes contain two. The catalytic domain consists of about 250 amino acids (Niels Peter Hundahl Moller et al. Protein tyrosine phosphatases (PTPs) as drug targets: Inhibitors of PTP-1B for the treatment of diabetes; Current Opinion in Drug Discovery & Development 3(5), 527-540 (2000)).
Protein-tyrosine phosphatases have been shown to be involved in various disorders. For instance PTP1B is generally viewed as being related to the insulin signalling and therefore is believed to be involved in disorders like diabetes mellitus type II.
In Nature, Vol. 5, January 2005, Tomas Mustelin et al presented a summary of what is known in respect of protein-tyrosine phosphatases and the immune response : A mouse having a mutation in SHP1 has provided a first example of an autoimmune disease caused by a defect in a PTP. The same is true for CD45 mutants as well as PEP (the mouse ortholog of LYP).
GLEPP-1 (also known as Glomerular epithelial protein 1, PTP-U2, PTPRO, PTP-oc, PTP-BK, PTP-Φ; GenBank Accession U20489) was discovered as a gene whose expression in Leukemia cell line U937 is overexpressed by Phorbol ester. The gene is expressed in macrophages, kidney, brain (Seimiya and Tsuruo 1993) and B-cells (Aguiar, Yakushijin et al. 1999).
Glomerular epithelial protein 1 (Glepp-1) is a receptor tyrosine phosphatase present on apical cell surface of the glomerular podocyte. The podocyte (visceral glomerular epithelial cell) is one of the major cell types responsible for the maintenance of the structure and function of the glomerular filter. Water, ions and small molecules from blood easily cross the filter while the passage of larger blood proteins (e.g. albumin) is minimized. Glcpp-1 has a single transmembrane domain, a single intracellular phosphatase domain and a large extracellular domain comprising fibronectin type III-like repeats
Glepp1 mutant mice are healthy and viable. These animals have only been studied for kidney morphology and functionality. While subtle morphological changes were observed, kidney functionality in the mutant mice is normal (Wharram, Goyal et al. 2000).
Work in macrophages shows that Glepp1 expression is regulated by CSF-1 (colony-stimulating factor). Glepp1 dephosphorylates paxillin and is required for cell motility (chemotaxis and extravasation; Pixley, Lee et al. 1995; Pixley, Lee et al. 2001).
Recent work shows that the Glepp1 gene is often hypermethylated and silenced in lung and colon cancers. (Mori, Yin et al. 2004; Motiwala, Kutay et al. 2004). However, no mention is made of increased tumor incidence in the Glepp1 knockout mice (Wharram, Goyal et al. 2000).

Summary of the invention

The present invention is related to the use of a Glepp-1 inhibitor for the manufacture of a medicament for the treatment of autoimmune and/or inflammatory disorders.

Detailed description of the invention

The following paragraphs provide defmitions of the various chemical moieties that make up the compounds according to the invention and are intended to apply uniformly through-out the specification and claims unless an otherwise expressly set out definition provides a broader definition.
"Glepp-1" refers to Glomerular epithelial protein 1 and has the following synonyms :, PTP-oc (rabbit), PTPRO, PTP-U2, PTP-U2L, PTP-U2S, PTP-phi.
"$C_1$-$C_6$-alkyl" refers to alkyl groups having 1 to 6 carbon atoms. This term is exemplified by groups such as methyl, ethyl, n-propyl, isopropyl, n-butyl, isobutyl, *tert*-butyl, n-pentyl, n-hexyl and the like.
"$C_1$-$C_8$-alkyl" refers to alkyl groups having 1 to 8 carbon atoms. This term is exemplified by groups such as methyl, ethyl, n-propyl, isopropyl, n-butyl, isobutyl, *tert*-butyl, n-pentyl, n-hexyl, n-heptyl, n-octyl, 2-ethylhexyl, 2-octyl,

3-octyl, isooctyl and the like.

"$C_1$-$C_{12}$-alkyl" refers to alkyl groups having 1 to 12 carbon atoms. This term is exemplified by groups such as methyl, ethyl, n-propyl, isopropyl, n-butyl, isobutyl, *tert*-butyl, n-pentyl, n-hexyl, n-heptyl, n-octyl, 2-ethylhexyl, 2-octyl, 3-octyl, isooctyl, n-nonyl, 2-nonyl, 3-nonyl, 4-nonyl, isononyl, n-decyl, isodecyl, 2-decyl, 3-decyl, n-undecyl, n-dodecyl and the like.

"$C_6$-$C_{15}$-alkyl" refers to alkyl groups having 6 to 15 carbon atoms. This term is exemplified by groups such as n-hexyl, 2-hexyl, 3-hexyl, n-heptyl, n-octyl, 2-ethylhexyl, 2-octyl, 3-octyl, isooctyl, n-nonyl, 2-nonyl, 3-nonyl, 4-nonyl, isononyl, n-decyl, isodecyl, 2-decyl, 3-decyl, n-undecyl, n-dodecyl and the like.

"$C_7$-$C_{15}$-alkyl" refers to alkyl groups having 7 to 15 carbon atoms. This term is exemplified by groups such as n-heptyl, n-octyl, 2-ethylhexyl, 2-octyl, 3-octyl, isooctyl, n-nonyl, 2-nonyl, 3-nonyl, 4-nonyl, isononyl, n-decyl, isodecyl, 2-decyl, 3-decyl, n-undecyl, n-dodecyl and the like.

"$C_8$-$C_{12}$-alkyl" refers to alkyl groups having 8 to 12 carbon atoms. This term is exemplified by groups such as n-octyl, 2-ethylhexyl, 2-octyl, 3-octyl, isooctyl, n-nonyl, 2-nonyl, 3-nonyl, 4-nonyl, isononyl, n-decyl, isodecyl, 2-decyl, 3-decyl, n-dodecyl and the like.

"Aryl" refers to an unsaturated aromatic carbocyclic group of from 6 to 14 carbon atoms having a single ring (*e.g.*, phenyl) or multiple condensed rings (*e.g*, naphthyl). Preferred aryl include phenyl, naphthyl, phenantrenyl and the like.

"$C_1$-$C_6$-alkyl aryl" refers to $C_1$-$C_6$-alkyl groups having an aryl substituent, including benzyl, phenethyl and the like.

"Heteroaryl" refers to a monocyclic heteroaromatic, or a bicyclic or a tricyclic fused-ring heteroaromatic group. Particular examples of heteroaromatic groups include optionally substituted pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadia-zolyl, 1,2,5-oxadiazolyl, 1,3,4-oxadiazolyl, 1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazoly, benzoxa-zolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

"$C_1$-$C_6$-alkyl heteroaryl" refers to $C_1$-$C_6$-alkyl groups having a heteroaryl substituent, including 2-furylmethyl, 2-thienylmethyl, 2-(1H-indol-3-yl)ethyl and the like.

"$C_2$-$C_6$-alkenyl" refers to alkenyl groups preferably having from 2 to 6 carbon atoms and having at least 1 or 2 sites of alkenyl unsaturation. Preferable alkenyl groups include ethenyl ($-CH=CH_2$), n-2-propenyl (allyl, $-CH_2CH=CH_2$) and the like.

"$C_2$-$C_6$-alkenyl aryl" refers to $C_2$-$C_6$-alkenyl groups having an aryl substituent, including 2-phenylvinyl and the like.

"$C_2$-$C_6$-alkenyl heteroaryl" refers to $C_2$-$C_6$-alkenyl groups having a heteroaryl substituent, including 2-(3-pyridinyl)vinyl and the like.

"$C_2$-$C_{12}$-alkenyl" refers to alkenyl groups preferably having from 2 to 12 carbon atoms and having at least 1 or 2 sites of alkenyl unsaturation. Preferable alkenyl groups include ethenyl ($-CH=CH_2$), n-2-propenyl (allyl, $-CH_2CH=CH_2$), a variety of butenyl isomers, crotonyl, isopentenyl, and the like.

"$C_2$-$C_{15}$-alkenyl" refers to alkenyl groups preferably having from 2 to 15 carbon atoms and having at least 1 or 2 sites of alkenyl unsaturation. Preferable alkenyl groups include ethenyl ($-CH=CH_2$), n-2-propenyl (allyl, $-CH_2CH=CH_2$), a variety ofbutenyl isomers, crotonyl, isopentenyl, and the like.

"$C_2$-$C_6$-alkynyl" refers to alkynyl groups preferably having from 2 to 6 carbon atoms and having at least 1-2 sites of alkynyl unsaturation, preferred alkynyl groups include ethynyl ($-C=CH$), propargyl ($-CH_2C\equiv CH$), and the like.

"$C_2$-$C_6$-alkynyl aryl" refers to $C_2$-$C_6$-alkynyl groups having an aryl substituent, including phenylethynyl and the like.

"$C_2$-$C_6$-alkynyl heteroaryl" refers to $C_2$-$C_6$-alkynyl groups having a heteroaryl substituent, including 2-thienylethynyl and the like.

"$C_2$-$C_{12}$-alkynyl" refers to alkynyl groups preferably having from 2 to 12 carbon atoms and having at least 1-2 sites of alkynyl unsaturation, preferred alkynyl groups include ethynyl ($-C=CH$), propargyl ($-CH_2C\equiv CH$), 6-heptynyl, 7-octynyl, 8-nonynyl, and the like.

"$C_2$-$C_{15}$-alkynyl" refers to alkynyl groups preferably having from 2 to 15 carbon atoms and having at least 1-2 sites of alkynyl unsaturation, preferred alkynyl groups include ethynyl ($-C\equiv CH$), propargyl ($-CH_2C\equiv CH$), 6-heptynyl, 7-octynyl, 8-nonynyl, and the like.

"$C_3$-$C_8$-cycloalkyl" refers to a saturated carbocyclic group of from 3 to 8 carbon atoms having a single ring (*e.g.*, cyclohexyl) or multiple condensed rings (*e.g.*, norbornyl). Preferred cycloalkyl include cyclopentyl, cyclohexyl, norbornyl and the like.

"$C_1$-$C_6$-alkyl cycloalkyl" refers to $C_1$-$C_6$-alkyl groups having a cycloalkyl substituent, including cyclohexylmethyl, cyclopentylpropyl, and the like.

"heterocycloalkyl" refers to a $C_3$-$C_8$-cycloalkyl group according to the definition above, in which 1 to 3 carbon atoms are replaced by hetero atoms chosen from the group consisting of O, S, NR, R being defined as hydrogen or $C_1$-$C_6$ alkyl. Preferred heterocycloalkyl include pyrrolidine, piperidine, piperazine, 1-methylpiperazine, morpholine, and the like.

"$C_1$-$C_6$-alkyl heterocycloalkyl" refers to $C_1$-$C_6$-alkyl groups having a heterocycloalkyl substituent, including 2-(1-pyrrolidinyl)ethyl, 4-morpholinylmethyl, (1-methyl-4-piperidinyl)methyl and the like.

"Carboxylic acid" refers to the group -C(O)OH.

"$C_1$-$C_6$-alkyl carboxy" refers to $C_1$-$C_6$-alkyl groups having a carboxy substituent, including 2-carboxyethyl and the like.

"Acyl" refers to the group -C(O)R where R includes H, "$C_1$-$C_6$-alkyl", "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "$C_2$-$C_6$-alkynyl heteroaryl", "$C_1$-$C_6$-alkyl cycloalkyl", "$C_1$-$C_6$-alkyl heterocycloalkyl".

"$C_1$-$C_6$-alkyl acyl" refers to $C_1$-$C_6$-alkyl groups having an acyl substituent, including 2-acetylethyl and the like.

"Aryl acyl" refers to aryl groups having an acyl substituent, including 2-acetylphenyl and the like.

"Heteroaryl acyl" refers to hetereoaryl groups having an acyl substituent, including 2-acetylpyridyl and the like.

"$C_3$-$C_8$-(hetero)cycloalkyl acyl" refers to 3 to 8 membered cycloalkyl or heterocycloalkyl groups having an acyl substituent

"Acyloxy" refers to the group -OC(O)R where R includes H, "$C_1$-$C_6$-alkyl", "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "$C_2$-$C_6$-alkynyl heteroaryl", "$C_1$-$C_6$-alkyl cycloalkyl", "$C_1$-$C_6$-alkyl heterocycloalkyl".

"$C_1$-$C_6$-alkyl acyloxy" refers to $C_1$-$C_6$-alkyl groups having an acyloxy substituent, including 2-(acetyloxy)ethyl and the like.

"Alkoxy" refers to the group -O-R where R includes "$C_1$-$C_6$-alkyl", "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "C2-C6-alkynyl heteroaryl", "$C_1$-$C_6$-alkyl cycloalkyl", "$C_1$-$C_6$-alkyl heterocycloalkyl".

"$C_1$-$C_6$-alkyl alkoxy" refers to $C_1$-$C_6$-alkyl groups having an alkoxy substituent, including 2-ethoxyethyl and the like.

"Alkoxycarbonyl" refers to the group -C(O)OR where R includes "$C_1$-$C_6$-alkyl", "$C_2$-$C_6$-alkenyl", "$C_2$-$C_6$-alkynyl", "$C_3$-$C_8$-cycloalkyl", "heterocycloallcyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "C2-C6-alkynyl heteroaryl", "$C_1$-$C_6$-alkyl cycloalkyl", "$C_1$-$C_6$-alkyl heterocycloalkyl".

"$C_1$-$C_6$-alkyl alkoxycarbonyl" refers to $C_1$-$C_6$-alkyl groups having an alkoxycarbonyl substituent, including 2-(benzyloxycarbonyl)ethyl and the like.

"Aminocarbonyl" refers to the group -C(O)NRR' where each R, R' includes independently hydrogen, "$C_1$-$C_6$-alkyl", "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "C2-C6-alkynyl heteroaryl", "$C_1$-$C_6$-alkyl cycloalkyl", "$C_1$-$C_6$-alkyl heterocycloalkyl".

"$C_1$-$C_6$-alkyl aminocarbonyl" refers to $C_1$-$C_6$-alkyl groups having an aminocarbonyl substituent, including 2-(dimethylaminocarbonyl)ethyl and the like.

"Acylamino" refers to the group -NRC(O)R' where each R, R' is independently hydrogen, "$C_1$-$C_6$-alkyl", "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "$C_2$-$C_6$-alkynyl heteroaryl", "$C_1$-$C_6$-alkyl cycloalkyl", "$C_1$-$C_6$-alkyl heterocycloalkyl".

"$C_1$-$C_6$-alkyl acylamino" refers to $C_1$-$C_6$-alkyl groups having an acylamino substituent, including 2-(propionylamino)ethyl and the like.

"Ureido" refers to the group -NRC(O)NR'R" where each R, R', R" is independently hydrogen, "$C_1$-$C_6$-alkyl", "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "$C_2$-$C_6$-alkynyl heteroaryl", "$C_1$-$C_6$-alkyl cycloalkyl", "$C_1$-$C_6$-alkyl heterocycloalkyl", and where R' and R", together with the nitrogen atom to which they are attached, can optionally form a 3-8-membered heterocycloalkyl ring.

"$C_1$-$C_6$-alkyl ureido" refers to $C_1$-$C_6$-alkyl groups having an ureido substituent, including 2-(N'-methylureido) ethyl and the like.

"Carbamate" refers to the group -NRC(O)OR' where each R, R' is independently hydrogen, "$C_1$-$C_6$-alkyl", "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "$C_2$-$C_6$-alkynylheteroaryl",

"$C_1$-$C_6$-alkyl cycloalkyl", "$C_1$-$C_6$-alkyl heterocycloalkyl".

"$C_1$-$C_6$-alkyl carbamate" refers to $C_1$-$C_6$-alkyl groups having a carbamate substituent.

"Amino" refers to the group -NRR' where each R, R' is independently hydrogen, "$C_1$-$C_6$-alkyl", "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "$C_2$-$C_6$-alkynyl heteroaryl", "$C_1$-$C_6$-alkyl cycloalkyl", "$C_1$-$C_6$-alkyl heterocycloalkyl", and where R and R', together with the nitrogen atom to which they are attached, can optionally form a 3-8-membered hetero-cycloalkyl ring.

"$C_1$-$C_6$-alkyl amino" refers to $C_1$-$C_6$-alkyl groups having an amino substituent, including 2-(1-Pyrrolidinyl)ethyl and the like.

"Ammonium" refers to a positively charged group -$N^+$RR'R", where each R, R',R" is independently, "$C_1$-$C_6$-alkyl", "$C_2$-$C_6$-alkenyl", "$C_2$-$C_6$-alkynyl", "$C_3$-$C_8$-cycloalkyl", "heterocycloalkyl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "C2-C6-alkynyl heteroaryl", "$C_1$-$C_6$-alkyl cycloalkyl", "$C_1$-$C_6$-akyl heterocycloalkyl", and where R and R', together with the nitrogen atom to which they are attached, can optionally form a 3-8-membered heterocycloalkyl ring.

"$C_1$-$C_6$-alkyl ammonium" refers to $C_1$-$C_6$-alkyl groups having an ammonium substituent, including 2-(1-pyrrolidinyl)ethyl and the like.

"Halogen" refers to fluoro, chloro, bromo and iodo atoms.

"Sulfonyloxy" refers to a group -$OSO_2$-R wherein R is selected from H, "$C_1$-$C_6$-alkyl", "$C_1$-$C_6$-alkyl" substituted with halogens, e.g., an -$OSO_2$-$CF_3$ group, "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "C2-C6-alkynyl heteroaryl", "$C_1$-$C_6$-alkyl cycloalkyl", "$C_1$-$C_6$-alkyl heterocycloalkyl".

"$C_1$-$C_6$-alkyl sulfonyloxy" refers to $C_1$-$C_6$-alkyl groups having a sulfonyloxy substituent, including 2-(methylsulfonyloxy)ethyl and the like.

"Sulfonyl" refers to group "-$SO_2$-R" wherein R is selected from H, "aryl", "heteroaryl", "$C_1$-$C_6$-akyl", "$C_1$-$C_6$-alkyl" substituted with halogens, *e.g.*, an -$SO_2$-$CF_3$ group, "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-akyl heteroaryl", "$C_2$-$C_6$-alkenyl aryl", "$C_2$-$C_6$-alkenyl heteroaryl", "$C_2$-$C_6$-alkynyl aryl", "C2-C6-alkynyl heteroaryl", "$C_1$-$C_6$-alkyl cycloalkyl", "$C_1$-$C_6$-alkyl heterocycloalkyl".

"$C_1$-$C_6$-alkyl sulfonyl" refers to $C_1$-$C_6$-alkyl groups having a sulfonyl substituent, including 2-(methylsulfonyl) ethyl and the like.

"Sulfinyl" refers to a group "-S(O)-R" wherein R is selected from H, "$C_1$-$C_6$-alkyl", "$C_1$-$C_6$-alkyl" substituted with halogens, *e.g.*, an -SO-$CF_3$ group, "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "C2-C6-alkynyl heteroaryl", "$C_1$-$C_6$-alkyl cycloalkyl", "$C_1$-$C_6$-alkyl heterocycloalkyl".

"$C_1$-$C_6$-alkyl sulfinyl" refers to $C_1$-$C_6$-alkyl groups having a sulfinyl substituent, including 2-(methylsulfinyl)ethyl and the like.

"Sulfanyl" refers to groups -S-R where R includes H, "$C_1$-$C_6$-alkyl", "$C_1$-$C_6$-alkyl" optionally substituted with halogens., *e.g* a -S-$CF_3$ group, "$C_2$-$C_6$-alkenyl", "$C_2$-$C_6$-akynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "C2-C6-alkynyl heteroaryl", "$C_1$-$C_6$-alkyl cycloalkyl", "$C_1$-$C_6$-alkyl heterocycloalkyl". Preferred sulfanyl group include methylsulfanyl, ethylsulfanyl, and the like.

"$C_1$-$C_6$-alkyl sulfanyl" refers to $C_1$-$C_6$-alkyl groups having a sulfanyl substituent, including 2-(ethylsulfanyl)ethyl and the like.

"Sulfonylamino" refers to a group -$NRSO_2$-R' where each R, R' includes independently hydrogen, "$C_1$-$C_6$-alkyl", "$C_2$-$C_6$-alkenyl", "$C_2$-$C_6$-alkynyl", "$C_3$-$C_8$-cycloalkyl", "heterocycloalkyl", "aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "C2-C6-alkynyl heteroaryl", "$C_1$-$C_6$-alkyl cycloalkyl", "$C_1$-$C_6$-alkyl heterocycloalkyl".

"$C_1$-$C_6$-alkyl sulfonylamino" refers to $C_1$-$C_6$-alkyl groups having a sulfonylamino substituent, including 2-(ethylsulfonylamino)ethyl and the like.

"Aminosulfonyl" refers to a group-$SO_2$-NRR' where each R, R' includes independently hydrogen, "$C_1$-$C_6$-alkyl", "$C_2$-$C_6$-alkenyl", "$C_2$-$C_6$-akynyl", "$C_3$-$C_8$-cycloalkyl", "heterocycloalkyl", aryl", "heteroaryl", "$C_1$-$C_6$-alkyl aryl" or "$C_1$-$C_6$-alkyl heteroaryl", "$C_2$-$C_6$-alkenyl aryl", "$C_2$-$C_6$-alkenyl heteroaryl", "$C_2$-$C_6$-alkynyl aryl", "C2-C6-alkynyl heteroaryl", "$C_1$-$C_6$-alkyl cycloalkyl", "$C_1$-$C_6$-alkyl heterocycloalkyl".

"$C_1$-$C_6$-akyl aminosulfonyl" refers to $C_1$-$C_6$-alkyl groups having an aminosulfonyl substituent, including 2-(cyclohexylaminosulfonyl)ethyl and the like.

"Substituted or unsubstituted": Unless otherwise constrained by the definition of the indi-vidual substituent, the above set out groups, like "alkyl", "alkenyl", "alkynyl", "aryl" and "heteroaryl" etc. groups can optionally be substituted with from 1 to 5 substituents selected from the group consisting of "$C_1$-$C_6$-alkyl", "$C_2$-$C_6$-alkenyl", "$C_2$-$C_6$-alkynyl", "cycloalkyl", "heterocycloalkyl", "$C_1$-$C_6$-alkyl aryl", "$C_1$-$C_6$-alkyl heteroaryl", "$C_1$-$C_6$-alkyl cycloalkyl", "$C_1$-$C_6$-alkyl heterocy-

cloalkyl", "amino", "ammonium", "acyl", "acyloxy", "acylamino", "aminocarbonyl", "alkoxycarbonyl", "ureido", "carbamate", "aryl", "heteroaryl", "sulfinyl", "sulfonyl", "alkoxy", "sulfanyl", "halogen", "carboxylic acid", trihalomethyl, cyano, hydroxy, mercapto, nitro, and the like. Alternatively, said substitution could also comprise situations where neighbouring substituents have undergone ring closure, notably when vicinal functional substituents are involved, thus forming, *e.g.*, lactams, lactons, cyclic anhydrides, but also acetals, thioacetals, aminals formed by ring closure for instance in an effort to obtain a protective group.

"Pharmaceutically acceptable salts or complexes" refers to salts or complexes of the below-specified compounds of Formula (I), (II), (III), (IV), (V), (VI) and (VII). Examples of such salts include, but are not restricted, to base addition salts formed by reaction of compounds of Formula (I), (II), (III), (IV), (V), (VI) and (VII) with organic or inorganic bases such as hydroxide, carbonate or bicarbonate of a metal cation such as those selected in the group consisting of alkali metals (sodium, potassium or lithium), alkaline earth metals (e.g. calcium or magnesium), ammonia, or with an organic primary, secondary or tertiary alkyl amine. Amine salts derived from methylamine, dimethylamine, trimethylamine, ethylamine, diethylamine, triethylamine, morpholine, N-Me-D-glucamine, N,N'-bis(phenylmethyl)-1,2-ethanediamine, tromethamine, ethanolamine, diethanolamine, ethylenediamine, N-methylmorpholine, procaine, piperidine, piperazine, arginine, choline, lysine and the like are contemplated being within the scope of the instant invention.

Also comprised are salts which are formed from to acid addition salts formed with inorganic acids (e.g. hydrochloric acid, hydrobromic acid, sulfuric acid, phosphoric acid, nitric acid, and the like), as well as salts formed with organic acids such as acetic acid, oxalic acid, tartric acid, citric acid, succinic acid, malic acid, fumaric acid, maleic acid, ascorbic acid, benzoic acid, tannic acid, palmitic acid, alginic acid, polyglutamic acid, naphthalene sulfonic acid, naphthalene disulfonic acid, and poly-galacturonic acid.

"Pharmaceutically active derivative" refers to any compound that upon administration to the recipient, is capable of providing directly or indirectly, the activity disclosed herein. The term "indirectly" also encompasses prodrugs which may be converted to the active form of the drug via endogenous enzymes or metabolism. Said prodrug is comprised of the active drug compound itself and a chemical masking group. Such masking group may be an ester moiety (e.g. obtained by masking a carboxylic acid or an hydroxy moiety of the compounds of Formula (I).

"Enantiomeric excess" (ee) refers to the products that are obtained by an asymmetric syn-thesis, i.e. a synthesis involving non-racemic starting materials and/or reagents or a syn-thesis comprising at least one enantioselective step, whereby a surplus of one enantiomer in the order of at least about 52% ee is yielded.

An "interferon" or "IFN", as used herein, is intended to include any molecule defined as such in the literature, comprising for example any types of IFNs mentioned in the above section "Background of the Invention". In particular, IFN-α, IFN-β and IFN-γ are included in the above definition. IFN-β is the preferred IFN according to the present invention. IFN-β suitable in accordance with the present invention is commercially available e.g. as Rebif® (Serono), Avonex® (Biogen) or Betaferon® (Schering).

The term "interferon-beta (IFN-beta or IFN-β)", as used herein, is intended to include fibroblast interferon in particular of human origin, as obtained by isolation from biological fluids or as obtained by DNA recombinant techniques from prokaryotic or eukaryotic host cells, as well as its salts, functional derivatives, variants, analogs and active fragments. Preferably, IFN-beta is intended to mean recombinant Interferon beta-1 a.

IFN-β suitable in accordance with the present invention is commercially available e.g. as Rebif® (Serono), Avonex® (Biogen) or Betaferon® (Schering). The use of interferons of human origin is also preferred in accordance with the present invention. The term interferon, as used herein, is intended to encompass salts, functional derivatives, variants, analogs and active fragments thereof.

The compounds used in the present invention also comprise their its tautomers, their geometrical isomers, their optically active forms as enantiomers, diastereoisomers and their racemate forms, as well as pharmaceutically acceptable salts and pharmaceutically active derivatives thereof. Preferred pharmaceutically acceptable salts of the compound used in the present invention are base addition salts formed by reaction of compounds with pharmaceutically acceptable bases like N-methyl-D-glucamine, tromethamine, lysine, arginine, choline, sodium, potassium or calcium salts of carbonates, bicarbonates or hydroxides.

The present invention relates to the use of Glepp-1 inhibitors for the treatment of an autoimmune and/or an inflammatory disorder alone or in combination with a further drug which is active in the treatment of autoimmune and/or an inflammatory disorders.

Any autoimmune and/or an inflammatory disorder is comprised by the present invention. Particular disorders comprised are : inflammatory bowel diseases, Crohn's disease, ulcerative colitis, collagenous colitis, lymphocytic colitis, diversion colitis irritable bowel syndrome, neuroinflammation including multiple sclerosis; Guillan Barré syndrome, chronic inflammatory polyncuropathy (CIPN), lung diseases including acute respiratory distress syndrome; joint and bone diseases including osteoarthritis and rheumatoid arthritis; liver diseases including liver fibrosis, cirrhosis and chronic liver disease; fibrotic diseases including, lupus, glomerulosclerosis, systemic sclerosis skin fibrosis, post-radiation fibrosis and cystic fibrosis; vascular pathologies including atherosclerosis, cardiomyopathy and myocardial infarction; restenosis; and degenerative diseases of the central nervous system including amyotrophic lateral sclerosis or inflammatory disorders of the skin including scleroderma and psoriasis.

In an embodiment, the compounds of the invention can be used in the treatment of inflammatory and/or autoimmune diseases, e.g. demyelinating diseases such as multiple sclerosis, alone or in combination with a co-agent useful in the treatment of autoimmune diseases.

A suitable co-agent for the treatment of a demyelinating disease is selected from the following compounds:

(a) Interferons, e. g. pegylated or non-pegylated interferons, e. g. administered by subcutaneous, intramuscular or oral routes, preferably interferon beta;

(b) Glatiramer, e. g. in the acetate form;

(c) Immunosuppressants with optionally antiproliferative/antineoplastic activity, e. g. mitoxantrone, methotrexate, azathioprine, cyclophosphamide, or steroids, e. g. methylprednisolone, prednisone or dexamethasone, or steroid-secreting agents, e. g. ACTH;

(d) Adenosine deaminase inhibitors, e. g. Cladribine;

Inhibitors of VCAM-1 expression or antagonists of its ligand, e. g. antagonists of the α4/β1 integrin VLA-4 and/or alpha-4-beta-7 integrins, e. g. natalizumab (ANTEGREN).

Rebif® (recombinant interferon-β) is the latest development in interferon therapy for multiple sclerosis (MS) and represents a significant advance in treatment. Rebif® is interferon (IFN)-beta 1a, produced from mammalian cell lines. It was established that interferon beta-1 a given subcutaneously three times per week is efficacious in the treatment of Relapsing-Remitting Multiple Sclerosis (RRMS). Interferon beta-1a can have a positive effect on the long-term course of MS by reducing number and severity of relapses and reducing the burden of the disease and disease activity as measured by MRI. The dosing of IFN-beta in the treatment of relapsing-remitting MS according to the invention depends on the type of IFN-beta used.

Further anti-inflammatory agents (in particular for demyelinating diseases such as multiple sclerosis) are described below :

A further anti-inflammatory agent is Teriflunomide which is described in WO 02/080897

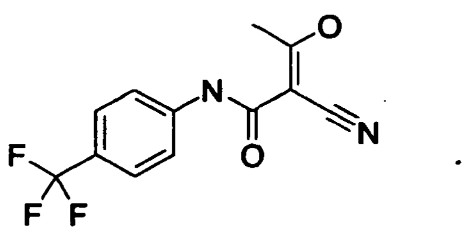

Still a further anti-inflammatory agent is Fingolimod which is described in EP-727,406 and WO 2004/028251.

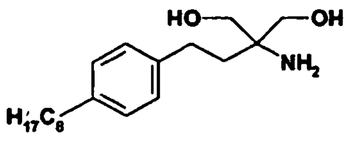

Still a further anti-inflammatory agent is Laquinimod which is described in WO 99/55678.

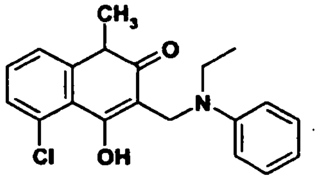

Still a further anti-inflammatory agent is Tensirolimus which is described in WO 02/28866.

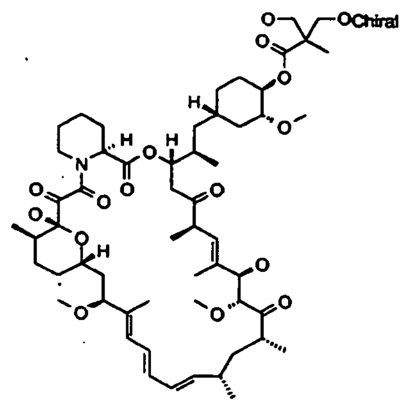

Still a further anti-inflammatory agent is Xaliprodene which is described in WO 98/48802.

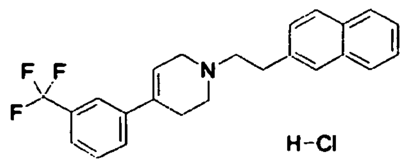

Still a further anti-inflammatory agent is Deskar Pirfenidone which is described in WO 03/068230.

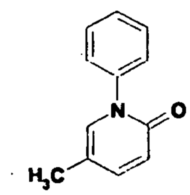

Still a further anti-inflammatory agent is the below benzothiazole derivative which is described in WO 01/47920.

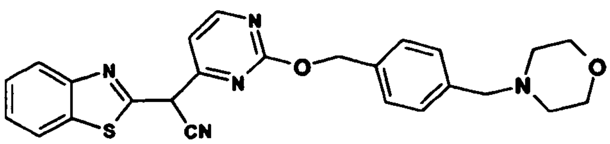

Still a further anti-inflammatory agent is the below hydroxamic acid derivative which is described in WO 03/070711.

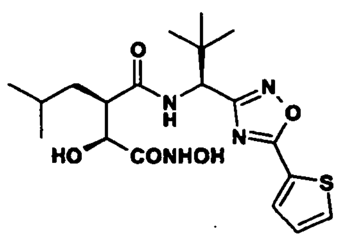

Still a further anti-inflammatory agent is MLN3897 which is described in WO2004/043965.

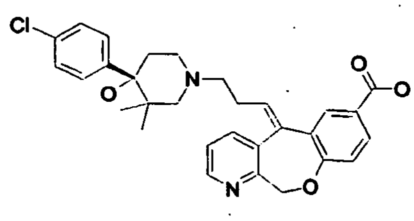

Still a further anti-inflammatory agent is CDP323 which is described in WO 99/67230.

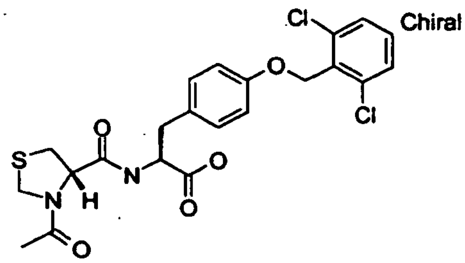

Still a further anti-inflammatory agent is Simvastatin which is described in WO 01/45698.

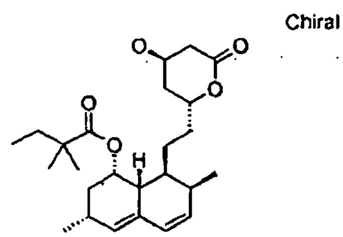

Still a further anti-inflammatory agent is Fampridine which is described in US-5540938.

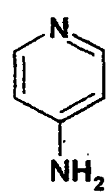

WO 96/33181, WO 2004/080377, WO 03/032999, WO 03/59871 and WO 2005/007616 also relate to anti-inflammatory agents.

In a first embodiment the Glepp-1 inhibitor which may be used is a carboxylic acid of Formula (I) :

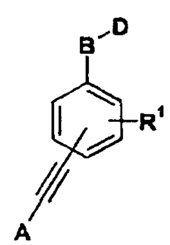

(I)

as well as its geometrical isomers, its optically active forms as enantiomers, diastereomers and its racemate forms, as

well as pharmaceutically acceptable salts and pharmaceutically active derivatives thereof.
Compounds according to Formula (I) as well as their preparation are described in WO 2005/097773.

The substituents A, B, D and $R^1$ within Formula (I) are defined as follows :

A is selected from the group consisting of aryl, $C_1$-$C_6$-alkyl aryl, In a preferred embodiment A is $C_4$-$C_6$ alkyl aryl, and in particular n-butylphenyl.

$R^1$ is selected from the group consisting of H, $C_1$-$C_6$-alkyl, $C_1$-$C_6$-alkoxy, halogen. In a specific embodiment $R^1$ is H.

B is either an amine selected from the group consisting of :

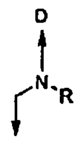

B1

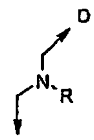

B2

or a carboxamide selected from the group consisting of :

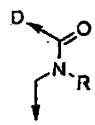

B3

or a sulfonamide selected from the group consisting of :

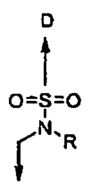

B6

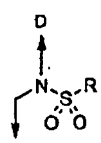

B7

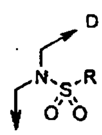

B8

or a urea moiety selected from the group consisting of :

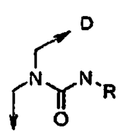

B9

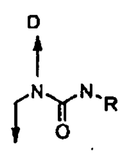

B20

wherein D is either selected from the group consisting of D1, D2, D3 herebelow

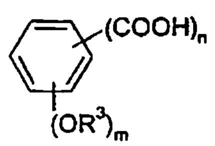 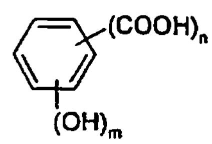 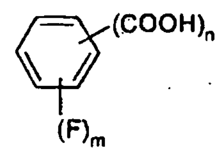

D1 D2 D3

with m being an integer selected from 0, 1 or 2 and n being an integer selected from 1 or 2 and $R^3$ is H or $C_1$-$C_6$ alkyl; or D4

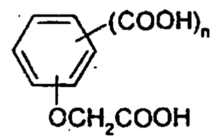

D4

with n being an integer selected from 0 or 1, and
wherein R is selected from the group consisting of $C_1$-$C_{12}$-alkyl or $C_1$-$C_8$ alkyl , $C_2$-$C_6$-alkenyl C2-$C_6$-alkynyl, $C_1$-$C_6$-alkoxy (including ethers or polyethers), $C_1$-$C_6$-alkyl amine, $C_1$-$C_6$-alkyl alkoxy, aryl, heteroaryl, saturated or unsaturated C3-C8- cycloalkyl, heterocycloalkyl, $C_1$-$C_6$-alkyl aryl (e.g. a benzyl group), $C_1$-$C_6$-alkyl heteroaryl, $C_2$-$C_6$-alkenyl aryl, $C_2$-$C_6$-alkenyl heteroaryl, $C_2$-$C_6$-alkynyl aryl, $C_2$-$C_6$-alkynyl heteroaryl, $C_1$-$C_6$-alkyl cycloalkyl, $C_1$-$C_6$-alkyl heterocycloalkyl, $C_2$-$C_6$-alkenyl cycloalkyl, $C_2$-$C_6$-alkenyl heterocycloalkyl, $C_2$-$C_6$-alkynyl cycloalkyl, $C_2$-$C_6$-alkynyl heterocycloalkyl.

Aryl or heteroaryl moities in R and A include phenyl, naphthyl, phenantrenyl, pyrrolyl, furyl, thienyl, imidazolyl, pyridyl, oxazolyl, isoxazolyl, thiazolyl, isothiazolyl, pyrazolyl, 1,2,3-triazolyl, 1,2,4-triazolyl, 1,2,3-oxadiazolyl, benzo(1,2,5) oxadiazolyl, 1,2,4-oxadiazolyl, 1,2,5-oxadiazolyl, 1,3,4-oxadiazolyl, tetrazolyl, 1,3,4-triazinyl, 1,2,3-triazinyl, benzopyrimidinyl, benzodioxolyl, benzofuryl, [2,3-dihydro]benzofuryl, isobenzofuryl, benzothienyl, indazolyl, benzotriazolyl, isobenzothienyl, indolyl, isoindolyl, 3*H*-indolyl, benzimidazolyl, benzothiazolyl, benzoxazolyl, pyridazinyl, pyrimidyl, quinolizinyl, quinazolinyl, phthalazinyl, quinoxalinyl, cinnolinyl, naphthyridinyl, quinolyl, isoquinolyl, tetrazolyl, 5,6,7,8-tetrahydroquinolyl, 5,6,7,8-tetrahydroisoquinolyl, purinyl, pteridinyl, xanthenyl, benzoquinolyl, oxolanyl, pyrrolidinyl, pyrazolidinyl, 2H-benzo[d] 1,3-dioxolenyl, indanyl, imidazolidinyl, 1,2,4-oxadiazolidinyl, 1,2,5-oxadiazolidinyl, 1,3,4-oxadiazolidinyl or isoxazolidinyl.

In a specific embodiment the aryl or heteroaryl moieties are : phenyl, pyridyl, pyrazolyl, benzodioxolyl, benzofuryl, benzothienyl, indazolyl.

Cycloalkyl moities in R and A include in particular cyclopentyl, or cyclohexyl groups.

A further specific embodiment consists in carboxylic acid of Formula (I), wherein B is either of

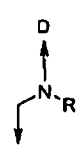 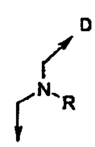 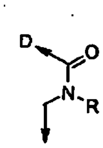

B1 B2 B3

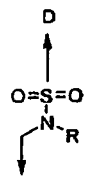

B6

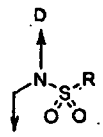

B7

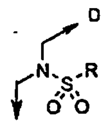

B8

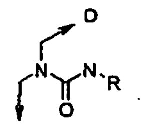

B9

and in a preferred embodiment B is either B1 or B2 or B3.

In another specific embodiment according to Formula (I) B is B20.

A further specific embodiment consists in carboxylic acid of Formula (I), wherein D is either of:

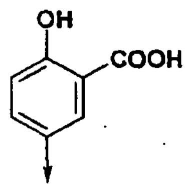

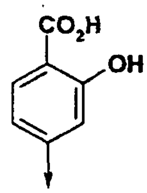

In a further specific embodiment D is

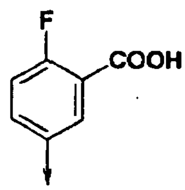

In still a further specific embodiment R is a $C_4$-$C_6$-alkyl, e.g. a hexyl group.

Preferred compounds of the invention are those of Formula (I), wherein A is a phenyl group substituted by a $C_1$-$C_4$-alkyl; B is either B1, B2, B3; R is a substituted or unsubstituted $C_4$-$C_6$-alkyl, a substituted or unsubstituted C3-C8-cycloalkyl, a substituted or unsubstituted $C_1$-$C_6$-alkyl cycloalkyl, e.g.- a methyl substituted by C3-C8-cycloalkyl (like a cyclopentyl or a cyclopropyl moiety); and D is

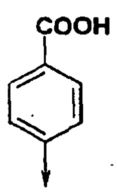

or

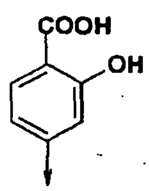

or

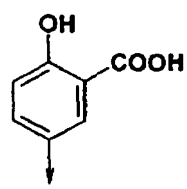

or

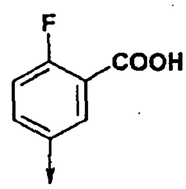

A further specific embodiment is related to compounds of Formula (Ia) wherein the substituent ethynyl-A is in the para-position as set out below :

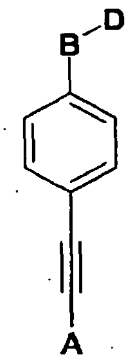

(Ia)

A specific embodiment consists in carboxylic acid of Formula (Ia) wherein A is an aryl moiety, in particular a substituted or unsubstituted phenyl group. A specific phenyl would be a phenyl being substituted by a $C_1$-$C_8$-alkyl, more preferably by a $C_1$-$C_4$-alkyl, , e.g. a butyl.

A further specific embodiment is related to compounds of Formula (Ib) and (Ic) wherein the substituent ethynyl-A is in the para-position or orto-positon as set out below

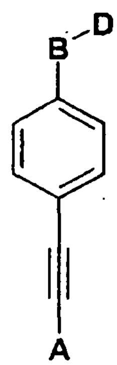

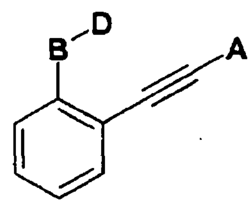

(Ib) (Ic)

wherein A is a $C_1$-$C_6$-alkyl aryl; B is B1, B3; R and D are as defined above.

Specific Glepp-1 inhibitors according to Formula (I) are selected from the group of :

5-[{4-[(4-butylphenyl)ethynyl]benzyl}(hexyl)amino]-2-hydroxybenzoic acid
5-[{4-[(4-butylphenyl)ethynyl]benzyl}(hexyl)amino]-2-fluorobenzoic acid
4-({{4-[(4-butylphenyl)ethynyl]benzyl}[2-(4-chlorophenyl)ethyl]amino}methyl)benzoic acid
5-[{4-[(4-butylphenyl)ethynyl]benzyl}(3-phenylpropyl)amino]-2-hydroxybenzoic acid
5-[{4-[(4-butylphenyl)ethynyl]benzyl}(1-naphthylmethyl)amino]-2-hydroxybenzoic acid
5-((4-tert-butylbenzyl){4-[(4-butylphenyl)ethynyl]benzyl}amino)-2-hydroxybenzoic acid
4-[{4-[(4-butylphenyl)ethynyl]benzyl}(hexyl)amino]-2-hydroxybenzoic acid
2-fluoro-5-{hexyl[4-(phenylethynyl)benzyl]amino}benzoic acid
5-[{4-[(4-butylphenyl)ethynyl]benzyl}(cyclopentylmethyl)amino]-2-fluorobenzoic acid
5-[{4-[(4-butylphenyl)ethynyl]benzyl}(3,3-dimethylbutyl)amino]-2-fluorobenzoic acid
5-[{4-[(4-butylphenyl)ethynyl]benzyl}(ethyl)amino]-2-fluorobenzoic acid
5-(hexyl{4-[(4-propylphenyl)ethynyl]benzyl}amino)-2-hydroxybenzoic acid
5-[{4-[(4-butylphenyl)ethynyl]benzyl}(pentyl)amino]-2-fluorobenzoic acid
5-[{4-[(4-butylphenyl)ethynyl]benzyl}(methyl)amino]-2-fluorobenzoic acid
5-[{4-[(4-butylphenyl)ethynyl]benzyl}(cyclopropylmethyl)amino]-2-fluorobenzoic acid
5-{butyl[4-(phenylethynyl)benzyl]amino}-2-fluorobenzoic acid
2-fluoro-5-[[4-(phenylethynyl)benzyl](propyl)amino]benzoic acid
2-fluoro-5-(hexyl{4-[(4-propylphenyl)ethynyl]benzyl}amino)benzoic acid
5-{{4-[(4-butylphenyl)ethynyl]benzyl}[(2-carboxycyclopropyl)methyl]amino}-2-fluorobenzoic acid

5-[{4-[(4-ethylphenyl)ethynyl]benzyl}(hexyl)amino]-2-fluorobenzoic acid
5-[{4-[(4-tert-butylphenyl)ethynyl]benzyl} (hexyl)amino]-2-fluorobenzoic acid
5-[{4-[(4-butylphenyl)ethynyl]benzyl}(isobutyl)amino]-2-fluorobenzoic acid
5-{[{4-[(4-butylphenyl)ethynyl]benzyl}(hexyl)amino]carbonyl}-2-hydroxybenzoic acid
5-{[{4-[(4-butylphenyl)ethynyl]benzoyl}(hexyl)amino]methyl}-2-hydroxybenzoic acid
5-{[{2-[(4-butylphenyl)ethynyl]benzyl}(hexyl)amino]carbonyl}-2-hydroxybenzoic acid
5-{[{4-[(4-butylphenyl)ethynyl]benzyl}(hexyl)amino]carbonyl}-2-fluorobenzoic acid
4-[{4-[(4-butylphenyl)cthynyl]benzyl}(3-cyclopentylpropanoyl)amino]-2-hydroxy-benzoic acid
5-[{4-[(4-butylphenyl)ethynyl]benzyl}(cyclohexylcarbonyl)amino]-2-hydroxybenzoic acid
5-[{4-[(4-butylphenyl)ethynyl]benzyl}(hexanoyl)amino]-2-hydroxybenzoic acid
5-((4-tert-butylbenzoyl){4-[(4-butylphenyl)ethynyl]benzyl}amino)-2-hydroxybenzoic acid
5-((biphenyl-4-ylcarbonyl){4-[(4-butylphenyl)ethynyl]benzyl}amino)-2-hydroxybenzoic acid
5-[{4-[(4-butylphenyl)ethynyl]benzyl}(3,3-dimethylbutanoyl)amino]-2-hydroxybenzoic acid
5-((1,3-benzodioxol-5-ylcarbonyl){4-[(4-butylphenyl)ethynyl]benzyl}amino)-2-hydroxybenzoic acid
5-([(benzyloxy)acetyl]{4-[(4-butylphenyl)ethynyl]benzyl}amino)-2-hydroxybenzoic acid
5-[{4-[(4-butylphenyl)ethynyl]benzyl}(4-hexylbenzoyl)amino]-2-hydroxybenzoic acid
5-((1-benzothien-2-ylcarbonyl){4-[(4-butylphenyl)ethynyl]benzyl}amino)-2-hydroxybenzoic acid
5-{[{4-[(4-butylphenyl)ethynyl]benzyl}(hexanoyl)amino]methyl}-2-hydroxybenzoic acid
(4-{[{4-[(4-butylphenyl)ethynyl]benzyl}(hexanoyl)amino]methyl}phenoxy)acetic acid
8-[{4-[(4-butylphenyl)ethynyl]benzyl}(3-cyclopentylpropanoyl)amino]-5,6,7,8-tedrahydronaphthalene-2-carboxylic acid
4-{[{4-[(4-butylphenyl)ethynyl]benzyl}(3-cyclopentylpropanoyl)amino]methyl}-benzoic acid
5-[{4-[(4-butylphenyl)ethynyl]benzyl}(3-cyclopentylpropanoyl)amino]-2-fluorobenzoic acid
5-[{4-[(4-butylphenyl)ethynyl]benzyl}(3,3-dimethylbutanoyl)amino]-2-fluorobenzoic acid
4-[{4-[(4-butylphenyl)ethynyl]benzyl}(cyclohexylcarbonyl)amino]-2-hydroxybenzoic acid
4-[{4-[(4-butylphenyl)ethynyl]benzyl}(hexanoyl)amino]-2-hydroxybenzoic acid
4-[{4-[(4-butylphenyl)ethynyl]benzyl}(3-cyclopentylpropanoyl)amino]-2-fluorobenzoic acid
4-[{4-[(4-tert-butylphenyl)ethynyl]benzyl}(3-cyclopentylpropanoyl)amino]-2-hydroxybenzoic acid
4-((3-cyclopentylpropanoyl){4-[(4-propylphenyl)ethynyl]benzyl}amino)-2-hydroxybenzoic acid
5-{[{4-[(4-butylphenyl)ethynyl]benzyl}(3-cyclopentylpropanoyl)amino]methyl}-2-hydroxybenzoic acid

A further aspect of the present invention consists in the use of a Glepp-1 inhibitor for the treatment of autoimmune and/or inflammatory disorders.

Still a further aspect of the present invention consists in Glepp-1 inhibitors for the treatment of autoimmune and/or inflammatory disorders.

Glepp-inhibitors may be identified by an assay methods described below (The Glepp-1 Binding Assay (in vitro assay)).

When employed as pharmaceuticals, Glepp-1 inhibitors of the present invention are typically administered in the form of a pharmaceutical composition. Hence, pharmaceutical compositions comprising a compound of Formula (I), (II), (III), (IV), (V), (VI) and (VII) and a pharmaceutically acceptable carrier, diluent or excipient therefore are also within the scope of the present invention. A person skilled in the art is aware of a whole variety of such carrier, diluent or excipient compounds suitable to formulate a pharmaceutical composition.

Such compositions can be prepared in a manner well known in the pharmaceutical art and comprise at least one active compound. Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The compounds of the invention, together with a conventionally employed adjuvant, car-rier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous use). Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

The pharmaceutical compositions of these inventions can be administered by a variety of routes including oral, rectal, transdermal, subcutaneous, intravenous, intramuscular, and intranasal. The compositions for oral administration can take the form of bulk liquid solutions or suspensions, or bulk powders. More commonly, however, the compositions are presented in unit dosage forms to facilitate accurate dosing. The term "unit dosage forms" refers to physically discrete

units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampoules or syringes of the liquid compositions or pills, tablets, capsules or the like in the case of solid compositions. In such compositions, the carboxylic acid according to the invention is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatine; an excipient such as starch or lactose, a disintegrating agent such as alginic acid, Primogel, or corn starch; a lubricant such as magnesium stearate; a glidant such as colloidal silicon dio-xide; a sweetening agent such as sucrose or saccharin; or a flavoring agent such as pepper-mint, methyl salicylate, or orange flavoring.

Injectable compositions are typically based upon injectable sterile saline or phosphate-buf-fered saline or other injectable carriers known in the art. As above mentioned, compounds of Formula (I), (II), (III), (IV), (V), (VI) and (VII) in such compositions is typically a minor component, frequently ranging between 0.05 to 10% by weight with the remainder being the injectable carrier and the like.

The above described components for orally administered or injectable compositions are merely representative. Further materials as well as processing techniques and the like are set out in Part 5 of Remington's Pharmaceutical Sciences, 20th Edition, 2000, Marck Publishing Company, Easton, Pennsylvania, which is incorporated herein be reference.

The compounds of this invention can also be administered in sustained release forms or from sustained release drug delivery systems. A description of representative sustained release materials can also be found in the incorporated materials in *Remington's Pharma-ceutical Sciences*.

**Example 1** : Biological assays

In the following an assay shall be presented that allow the identification of Glepp-1 inhibitors. Also, example assays in support of the the anti-inflammatory effect of Glepp-1 inhibitors shall be presented.

The compounds of Formula (I), (II), (III), (IV), (V), (VI) and (VII), may be subjected to the following assays:

(1) Glepp-1 binding assay (in vitro)

(2) In vitro assay(s): Cell functional effect. Effect on MCP-1-induced chemotaxis on THP-1 cells

(3) Lymphocyte recruitment by thioglycollate (in vivo model for inflammation)

(4) Collagen induced arthritis (CIA) in mice (in vivo model for inflammation)

(5) Contact hypersensitivity (CHS is a T cell-mediated model of skin inflammation)

(6) EAE (in vivo model for MS)

(7) Ulcerative colitis

**(1) The Glepp-1 Binding Assay (in vitro assay)**

Assays for the determination of the PTP inhibitory activity of test compounds are well known to a person skilled in the art. An example of such an assay is described below :

The PTP Enzyme Assay aims at determining the extent of inhibition of Glepp-1 in the presence of a test compound. The inhibition is illustrated by $IC_{50}$ values which denote the concentration of test compound necessary to achieve an inhibition of 50% of Glepp-1 using the following concentration of the Glepp-1 substrate DiFMUP :
- 30 μM DiFMUP for GLEPP-1.

a) PTPs cloning

The cloning and expression of the catalytic domain e.g. of Glepp-1, may be performed as described in S. Wächli et al. : J. Biol. Chem. 2000, 275(13), pp 9792-9796.

b) Materials and Methods

The DiFMUP assay allows to follow the dephosphorylation of DiFMUP (6,8-DiFluoro-4-MethylUmbelliferyl Phosphate) - which is the Glepp-1 substrate - mediated by Glepp-1 into its stable hydrolysis product, i.e. DiFMU (6,8-difluoro-7-hydroxy coumarin). Due to its rather low pKa and its high quantum yield, DiFMU allows to measure both acidic and alkaline phosphatase activities with a great sensitivity.

Assays were performed in a 96 well plate format, using the catalytic core of a human recombinant Glepp-1 as the enzyme and 6,8-Difluoro-4-MethylUmbelliferyl Phosphate (DiFMUP, Molecular Probes, D-6567) as a substrate. Compounds to be tested were dissolved in 100% DMSO at a concentration of 2 mM. Subsequent dilutions of the test compounds (to yield a concentration of 100, 30, 10, 3, 1,0.3, 0.1, 0.03, 0.01, 0.001 μM) were performed in 60 % DMSO manually. 8 μl of diluted compound or vehicle (60% DMSO = control) was distributed to a black Costar 96 well plate. 42μl of human recombinant Glepp-1 enzyme diluted in assay buffer (20mM Tris HCl pH 7.5, 0.01% IGEPAL CA-630, 0.1mM ethylenediaminetetracetic acid, 1mM DL-Dithiothreitol) can be added to the dilutions of compound or vehicule (distributed to a black Costar 96 well plate), followed by 50μl of DiFMUP diluted in the assay buffer. The reaction ran for 30 minutes at room temperature before reading the fluorescence intensity (integral or intensity) on a Perkin-Elmer Victor 2 spectrofluorimeter (excitation of 6,8-difluoro-7-hydroxy coumarin is at 355nm, the emission at 460 nm, for 0.1s). The percentage of inhibition is determined by measuring the relative fluorescence ion absence of a test compound (PTP inhibitor), i.e. with the solvent alone (5% DMSO). The $IC_{50}$ values for inhibition were determined in triplicates.

The tested compounds according to Formula (I) display an inhibition (illustrated by $IC_{50}$ values) with regard to Glepp-1 of preferably less than 20 μM, more preferred less than 5 μM.

Table 1 : $IC_{50}$ values for test compounds (Glepp-1 Inhibitors)

| Glepp-1 Inhibitor (Test Compound) | Inhibition ($IC_{50}$) |
|---|---|
| (4E)-4-[(5-bromo-2-furyl)methylene]-1-(4-iodophenyl)pyrazolidine-3,5-dione | < 1 μM |
| 4-cyano-N-(4-{[(2Z)-2-(5-iodo-2-oxo-1,2-dihydro-3H-indol-3-ylidene)hydrazino]carbonyl} phenyl)benzamide | < 1 μM |
| ({{4-[(4-hexylphenyl)ethynyl]benzyl} [4-(trifluoromethyl)benzyl]-amino}-(oxo)acctic acid | < 1 μM |
| 5-((4-dcc-1-ynylbenzyl){(2*E*)-3-[3-(trifluoromethyl)phenyl]prop-2-enoyl}amino)2-hydroxybenzoic acid | < 1 μM |
| (4-{[[(2-{4-[(octylamino)carbonyl]phenyl}-1,3-thiazol-4-yl)methyl](3-phenylpropanoyl) amino]methyl}phenoxy)acetic acid | < 1 μM |
| 5-[{4-[(4-butylphenyl)ethynyl]benzyl}(hexyl)amino]-2-hydroxy-benzoic acid | < 1 μM |
| 5-{[{4-[(4-butylphenyl)ethynyl]benzyl}(hexyl)amino]carbonyl}-2-hydroxybenzoic acid | < 1 μM |

**(2) in vitro assay(s): Cell functional effect. *In vitro* chemotaxis.**

The assay was performed as follows: After starvation of THP1 cells in serum free medium for 3 hours, $10^7$ cells/ml in medium containing 0.5% BSA, and inhibitors, when indicated. 100 μl of cells were applied to the top chamber (5 μm pore size transwell plates, COSTAR, New York) and 600 μl of medium containing MCP-1/CCL2, in the absence or presence of test compounds, were added to the lower chamber. After incubation at 37°C and 5% of $CO_2$ for 3 hours, cells that passed through the membrane were collected and counted in a Beckman Coulter® AcT 5diffTM.

Test Compounds ({{4-[(4-hexylphenyl)ethynyl]benzyl}[4-(triftuoromethyl)-benzyl]amino}-(oxo)acetic acid, 5-[{4-[(4-butylphenyl)ethynyl]benzyl}(hexyl)amino]-2-hydroxybenzoic acid, 5-{[{4-[(4-butylphenyl)ethynyl]benzyl}(hexyl) amino]carbonyl}-2-hydroxybenzoic acid) block chemotaxis of lymphocytes with $EC_{50}$ between 1 and 15 uM.

*In vitro* chemotaxis using human monocytes:

The assay was performed as follows:

Material:

- Medium (RPMI 1640 without phenol red), containing L-glutamine, 0.3% DMSO and 2% foetal calf serum.
- 96-well filter plates (Neuro Probe Inc, ChemoTX #101-5) with a 5 micron pore size.

- 96-well funnel plates (Neuro Probe Inc, FP1).
- Black 96-well reader plates (Costar # 3915).
- Chemokine (MCP-1, R&D systems), use at 1 micro molar.
- CyQUANT Cell Proliferation Assay Kit (Molecular Probe Inc. # C7026).
- Buffy coats for preparation of monocytes by MACS sorting.
- autoMACS® purification kit (Miltenyi Biotec # 130-091-153).

Method:

- Prepare monocytes from a single buffy coat by negative depletion using the autoMACS® magnetic cell sorting kit for preparation of 'untouched' monocytes.
- Incubate monocytes ($1 \times 10^6$ cells/ml) with test compounds to chosen final concentration(s) for **20 minutes** at 37° C.
- Prepare chemotaxis plate: add chemokine to the lower chambers of the chemotaxis wells (during this time it is advisable to place the filter to one side, inside the plate cover, and to avoid touching the filter).
- Add the test compounds, where appropriate, to the lower chambers of the chemotaxis wells with the chemokine (N.B. the compounds are thus present in both the upper and lower chambers during the chemotaxis).
- Distribute the monocytes (20,000 cells per well in a total volume of 20 micro litres) on to the top of the plate filter taking care to avoid air bubbles. Replace plate lid.
- Incubate at 37°C for 2 hours.
- Carefully remove the plate from the incubator and remove the lid. Wash the filter with 10 ml of PBS in order to remove cells that have not migrated. This is achieved by using a 10ml pipette and inclining the plate at 45°. Without touching the filter itself, let the PBS run over the filter by tapping the pipette tip gently against the metal border.
- Transfer the migrated cells from the transmigration plate to a black reader plate with the aid of the funnel assembly and by centrifuging at 2000 rpm for 2 minutes.
- Place an adhesive plate cover onto the plates and leave at -80°C overnight.
- The next day, defrost the plates and add CyQUANT.
- Read the plates in a Wallac Victor Fluorescent Counter (excitation at 480 nm, emission at 520 n.

**(3) Lymphocyte recruitment by thioglycollate (in vivo model for inflammation)**

The following assay aims at determining the anti-inflammatory effect of Glepp-1 inhibitors in a model of lymphocyte recruitment by a thioglycollate (i.e. an inflammatory agent) challenge of mice, *in vivo.*

The assay was performed as follows :
A total of 6 C*H mice (Breeding Janvier about 8-9 weeks; obtained from IFFACREDO, l'Arbreste, France) were used. 2 groups, each consisting of 6 animals were formed :

- Group 1 : The animals were administered *(per os)* a dose of 30 mg/kg of Dexamethasone (positive control).
- Group 2 : The animals were administered *(per os)* a dose of 30 mg/kg of Glepp-1 inhibitor solubilized in the vehicle.
- Group 3 : Negative control (vehicle)

- Group 4 : Sham or baseline: the animals, receive the vehicle of the GLEPP and saline ("vehicle" of Thioglycollate).

*Experimental Protocol:*

8-9 weeks old female C3H mice were fasted during 18 hours. 15 minutes prior the intraperitoneal injection of thioglycollate (1.5%, 40 ml/kg), the mice were treated orally with a Glepp-1 inhibitor. Control mice received CMC/Tween as vehicle (10 ml/kg). The mice were then sacrificed by $CO_2$ inhalation and the peritoneal cavity was washed two times with 5 ml of ice-cold PBS/1 mM EDTA. The lavages were done 4hrs or 48 hrs after thioglycollate challenge to evaluate neutrophils or macrophages recruitment, respectively. The white blood cells (neutrophils, lymphocytes or macrophages) were counted using a Beckman Coulter ® A^cT 5diff™. Dexamethasone was used as reference drug.

Table 2 : Reduction of Neutrophils, Lymphocytes and Macrophages in blood (mice) following treatment with Glepp-1 inhibitor

| Test Compound Glepp-1 Inhibitor | Animals per Group | Decrease in blood Neutrophils | ± SEM | Decrease in blood lymphocytes | ± SEM | Decrease in blood Macrophages | ± SEM |
|---|---|---|---|---|---|---|---|
| {{4-[(4-hexylphenyl) ethynyl ]benzyl}[4-(trifluoromethyl) benzyl] amino}-(oxo) acetic acid | 6 | 27 | 5 | 29 | 5 | 27 | 7 |
| 5-[{4-[(4-butylphenyl) cthynyl] benzyl}(hexyl)amino ]-2-hydroxybenzoic acid | 6 | 44 | 4 | 47 | 4 | 50 | 6 |
| 5-{[{4-[(4-butylphenyl) ethynyl] benzyl}(hexyl)amino ] carbonyl}-2-hydroxy-benzoic acid | 6 | 41 | 6 | 41 | 5 | 62 | 3 |
| ([1-(3-chlorophenyl)-1-methylethyl]{4-[(4-hexylphenyl) ethynyl ]benzyl} amino)(oxo)acetic acid | 6 | 25 | 6 | 39 | 4 | 52 | 12 |
| {[4-(11-fluoroundec-1-yn-1-yl)benzyl][4-(trifluoromethyl) benzyl]amino}(oxo)acetic acid | 6 | 16 | 19 | 24 | 12 | 36 | 8 |

(continued)

| Test Compound Glepp-1 Inhibitor | Animals per Group | Decrease in blood Neutrophils | ± SEM | Decrease in blood lymphocytes | ± SEM | Decrease in blood Macrophages | ± SEM |
|---|---|---|---|---|---|---|---|
| {{4-[(4-hexylphenyl) ethynyl ]phenyl}[4-(trifluoromethyl) benzyl]amino}(oxo)acetic acid | 6 | 15 | 7 | 21 | 9 | 32 | 6 |
| 4-[{4-[(4-butylphenyl) ethynyl] benzyl}(hexyl)amino ]-2-hydroxybenzoic acid | 6 | 45 | 3 | 23 | 8 | 65 | 9 |
| 5-[{4-[(4-butylphenyl) ethynyl] benzyl}(cyclopentyl methyl)amino]-2-fluorobenzoic acid | 6 | 36 | 9 | 23 | 4 | 37 | 4 |
| 2-fluoro-5-(hexyl {4-[(4-propylphenyl) ethynyl]benzyl} amino)benzoic acid | 6 | -6 | 9 | 4 | 6 | 26 | 7 |
| 5-[{4-[(4-tert-butylphenyl) ethynyl] benzyl}(hexyl)amino ]-2-fluorobenzoic acid | 6 | 36 | 7 | 13 | 12 | 42 | 9 |
| 5-{[{4-[(4-butylphenyl) ethynyl] phenyl}(hexyl)amino ] methyl}-2-fluorobenzoic acid | 6 | 28 | 6 | 8 | 8 | 32 | 2 |
| (SEM = Standard Error of the Mean) | | | | | | | |

**(4) Collagen induced arthritis (CIA) in mice (in vivo model for inflammation)**

The following assay aims at determining the anti-inflammatory effect of Glepp-1 inhibitors in a model of collagen induced arthritis in mice, *in vivo*.

The assay was performed as follows:

Induction of chronic polyarthritis in certain rodent strains can be provoked by two injections of heterologous type II collagen (collagen-induced arthritis; CIA) when mixed with an adjuvant oil (1-3). Collagen-induced arthritis (CIA) is viewed as a validated animal model of rheumatoid arthritis. In this model, a chronic peripheral arthritis is elicited by intradermal injection of homologous or heterologous (bovine, chicken) type II collagen (CII) in adjuvant into rats or mice. In rodents, susceptibility to CIA has been shown to be primarily controlled by MHC genes. Evidence has accumulated that CIA is dependent upon T cell activation, including the T cell proliferative response to mouse CII in immunized mice, the successful adoptive transfer of the disease with immune spleen cells, and the resistance of athymic nude animals to the induction of the pathology (1-2).

Animals : Mice; Strain: DBA/1j (Charles River, Calco, Italy); Sex: Male; Age: 8 to 9 weeks.
Acclimatisation: Minimum of five days in the laboratory animal house where the experiment will take place.
Housing and feeding: the mice will be kept under standard laboratory conditions (non specific pathogen free) with ad libitum food and water.

Induction of CIA:

Murine type II CIA may be induced as described elsewhere (4-5). Positive control drugs such as dexamethazone and specific TNF-alpha inhibitors are equally effective in chicken and bovine type II C- induced arthritis. Briefly, chicken CII is dissolved in 0.01 M acetic acid at a concentration of 2 mg/ml by stirring overnight at 4°C. CFA is prepared by adding Mycobacterium tuberculosis H37Ra (Difco, Detroit, MI)) at a concentration of 5 mg/ml. Before injection CII are emulsified with an equal volume of CFA.
To induce CIA, the mice arc injected intradermally at the base of the tail with 100 ul of an emulsion containing 100 ug of CII. On day 21, a second injection of CII in CFA is administered.

Therapeutic treatment

For the therapeutic part of the study 5 groups of mice are created with animals presenting a clinical score ≥ 1 and that will be treated for 10 days as follows:

Group 1: test compound at 15 mg/kg p.o. twice a day
Group 2: test compound at 30 mg/kg p.o. twice a day
Group 3: test compound at 45 mg/kg p.o. twice a day
Group 4: mice treated with vehicle (0.5%Methocel A4M/0.25% Tween-20 in water)
Group 5: dexamethasone, 0.3 mg/kg as positive control group

Each group consists of 10 mice. Animals will be sacrificed after the last day of treatment.
Clinical assessment of CIA: Mice are evaluated daily for arthritis according to a macros-copic scoring system (3-4): 0= no signs of arthritis; 1= swelling and/or redness of the paw or 1 digit; 2= involvement of 2 joints; 3= involvement of > 2 joints, and 4= severe arthritis of the entire paw and digits. An arthritis index is calculated for each mouse by summing the scores for the individual paws.
Clinical severity is determined by quantitating changes in paw volume. Plethysmometry will be used to quantitate volume of both hindpaws.
Assessment of arthritis damage: The mice are sacrificed on day 28 post immunization. Blood is collected by intracardiac puncture and paws and knees are removed and fixed in 10% formalin for histologic examination by an observer unaware of the treatment regime of the mice. Histologic changes are examined by microscopy of both formalin-fixed paws and knees. Paws will be trimmed placed in decalcifying solution for 24 hours, embedded in paraffin, sectioned at 5 mcm, stained with hematoxilin and eosin and studied using light microscopy (4-5).
The following morphologic criteria are used: 0= no damage; 1= edema; 2 = presence of inflammatory cells, and 3= bone resorption (4-5)
The histologic assessment of articular cartilage erosion is performed by eosin hematoxylin staining using the same criteria for assessing with safranin staining, as described below.
Articular cartilage erosion: The assessment is based on a numerical score, increasing with the severity and extension of the lesion. These numbers refer to the overall assessment of the whole section:

0 = no erosion; articular cartilage intact
1-2 = localized articular cartilage erosion
3 = more extended erosions of the articular cartilage
4 = diffuse and massive destruction of the articular cartilage

The histological parameters are measured in all the groups of prophylactically-treated mice.

Blood sampling

In case of ameliorative findings during the clinical observations period, two hours after the last treatment, blood samples is collected from each animal (before the sacrifice) of the treated groups.

**(5) Contact hypersensitivity (CHS is a T cell-mediated model of skin inflammation)**

The following assay aims at determining the anti-inflamnatory effect of Glepp-1 inhibitors in a model of skin inflammation in mice, *in vivo*.

The assay was performed as follows :

Material:

- Female Balb/C mice between 8 wks and 12 weeks of age.
- Solvent: Acetone / olive oil mix (4:1)
- Hapten: DNFB (Dinitrofluorobenzene, Sigma#D1529).
- Sensitizing solution: 0.5% DNFB in solvent, freshly prepared.
- Challenging solution: 0.2% DNFB in solvent, freshly prepared.
- Caliper (Mitutoyo, Urdorf, Switzerland)
- Isofluran

Methods

Day 0: Sensitization of CHS:

For the induction of CHS groups of 72 mice are painted on the shaved back once with 35 μl of 0.5 % DNFB in acetone/olive oil (4:1). 6 mice serve as negative control (unsensitized animals)

Day 5: Elicitation of CHS:

Sensitized and unsensitized control animals are challenged (CH) on day +5 by appling 10 μl of 0.2% DNFB in acetone/olive oil (4:1) to each side of the right ears. As control, the left ear will be painted with an identical amount of the hapten vehicle.

1. n=12 Dosed with 0.5% Methocel A4M/0.25% Tween-20 in $dH_20$ p.o. 1 h before CH
2. n=12 Dosed with 1 mg/kg of test compound p.o. 1 h before CH
3. n=12 Dose with 3 mg/kg of test compound p.o. 1 h before CH
4. n=12 Dose with 10 mg/kg of test compound p.o. 1 h before CH
5. n=12 Dose with 30 mg/kg of test compound p.o. 1 h before CH
6. n=12 Dose with 1 mg/kg of Methotrexate s.c. 1 hour before CH

Right ears are partly removed and fixed in formaline and partly fixed in liquid nitrogen (for cytokine determination).

Day 6: Evaluation of ear swelling (basic read out)

Ear thickness of both ears is measured at three point each, ear swelling is evaluated according to the following formula:

$$[(T_n - T_5) \text{ right ear}] - [(T_n - T_5) \text{ left ear}]$$

where $T_n$ and $T_5$ represent values of averaged ear thickness at day n of investigation and at day 5 pre challenge, respectively.

**(6) EAE (in vivo model for MS)**

The following assay aims at determining the anti-inflammatory effect of Glepp-1 inhibitors in a model of EAE, *in vivo*.

The assay was performed as follows:

Effect of preventive treatment of a GLEPP inhibitor on a mouse chronic EAE (experimental autoimmune encephalomyelitis).

Materials

Mice: C57 BL/6N colony was supplied by Charles River Italia (Calco, Lecco, Italy).
With acclimation: At least 5 days before the study is initiated. In this period the animals will be observed daily to ascertain their fitness for the study.
Age of About 8-week old; 18-22 g.

Housing:

- 10 animals/cage in air-conditioned rooms.
- Temperature: 22°C ± 2
- Relative humidity: 55% ± 10
- Air changes: about 15-20/hour filtered on HEPA 99.99%.
- Light: 12 hour cycle (7 a.m. - 7 p.m.)
- Cage: Makrolone® cage 42.5x26.6x15h each fitted with a stainless steel cover-feed rack. A grill is inserted on the cage bottom. The waste that drops through the grill onto the cage bottom is periodically disposed of.

Study design

The study will involve 5 groups of 13 animals each. All the groups are assigned to the following treatments:

| Group | Test Substance | Dose | Administration volume/rate | Administration route | Treatment period | Frequency |
|---|---|---|---|---|---|---|
| 1 | Methocel 4C/ Tween (0.25%) in water | - | 10 mL/kg | p.o. | 21 days | Bid |
| 2 | Test Compound | 3 mg/kg | 10 mL/kg | p.o. | 21 days | Bid |
| 3 | Test Compound | 10 mg/kg | 10 mL/kg | p.o. | 21 days | Bid |
| 4 | Test Compound | 30 mg/kg | 10 mL/kg | p.o. | 21 days | Bid |

Vehicle: Methocel 4C/Tween (0.25%) in water will be used to dilute the test compound (a Glepp inhibitor) to the appropriate concentration.
Administration route: A test compound (a Glepp inhibitor) at the dose of 3, 10 and 30 mg/kg will be administered p.o. in a volume of 10 mL/kg. Group 1 will be dosed p.o. with vehicle (Methocel 4C/Tween (0.25%) in water) in a volume of 10 mL/kg.
The curative treatment will start at day 7 post immunization and will be continued for 21 days.

Clinical observations:

Starting from day 7 post-immunization the animals will individually be examined for the presence of paralysis by means of a clinical score as follows:

0 = no sign of disease
0.5 = partial tail paralysis
1 = tail paralysis
1.5 = tail paralysis + partial unilateral hindlimb paralysis
2 = tail paralysis + hindlimb weakness or partial hindlimb paralysis
2.5 = tail paralysis + partial hindlimb paralysis (lowered pelvi)
3 = tail paralysis + complete hindlimb paralysis
3.5 = tail paralysis + complete hindlimb paralysis + incontinence
4 = tail paralysis + hindlimb paralysis + weakness or partial paralysis of forelimbs

5 = moribund or dead

**(7) Dextran Sodium Sulfate-induced ulcerative colitis in mice**

**The assay was performed as follows:**

Ulcerative colitis (UC) was induced in female mice (Balb/c, 20-22g, Elevage Janvier) by Dextran Sodium Sulfate (DSS 4%) administered in drinking water. The mice had free access to DSS during 7 days. Body weight was determined daily. The severity of the UC was assessed by a clinical score (range 0 to 4) estimating the constituency of the stool (0= firm, 1= loose, 2= diarrhea) and the presence of blood (0= no blood, 1= occult blood, 2= gross rectal bleeding). Seven days after the induction of the disease, the animals were sacrificed. The length and the weight of the colon were determined and the ratio Weight / Length / 100 g body weight was calculated. The spleen weight was also determined.

Compound of the invention (10, 30 & 100 mg/kg, po) was suspended in 0.5% methocel / 0.25% tween 20 and was administered twice daily at days 3, 4, 5 and 6 after the induction of the UC. Sulfasalazine (200 mg/kg, po) was used as reference compound.

**Results**

5-[{4-[(4-butylphenyl)ethynyl]benzyl}(hexyl)amino]-2-hydroxybenzoic acid (10, 30 & 100 mg/kg, po) prevented significantly the colon reduction, estimated by the Weight / Length ratio, (-22%*, - 28%** & -35%***, respectively).

The splenomegaly was significantly reduced (-31%* & -65%***) and the clinical score was improved (-29% to -54% & -21% to -54%) when 5-[{4-[(4-butylphenyl)ethynyl]benzyl}-(hexyl)amino]-2-hydroxybenzoic acid was administered at the dose of 30 & 100 mg/kg, po, respectively.

The body weight loss was also prevented by 5-[{4-[(4-butylphenyl)ethynyl]-benzyl}(hexyl)amino]-2-hydroxybenzoic acid (10, 30 & 100 mg/kg, po).

5-{[{2-[(4-butylphenyl)ethynyl]benzyl}(hexyl)amino]carbonyl}-2-hydroxybenzoic acid (10& 30mg/kg, po) prevented significantly the colon reduction, estimated by the Weight / Length ratio, (-47%***& -50%***, respectively).

The splenomegaly was significantly reduced (-25%* & -45%**) and the clinical score was improved (-9% to -29% & -14% to -29%) when 5-{[{2-[(4-butylphenyl)ethynyl]benzyl}(hexyl)amino]carbonyl}-2-hydroxybenzoic acid was administered at the dose of 10 & 30 mg/kg, po, respectively.

The body weight loss was also prevented by 5-{[{2-[(4-butylphenyl)ethynyl]benzyl}(hexyl)amino]carbonyl}-2-hydroxybenzoic acid (10 & 30 mg/kg, po).

*: $p < 0.05$, **: $p < 0.01$, ***: $p < 0.001$ (drug treated groups versus vehicle treated group).

<u>List of references:</u>

- Aguiar, R. C., Y. Yakushijin, S. Kharbanda, S. Tiwari, G. J. Freeman and M. A. Shipp (1999). "PTPROt: an alternatively spliced and developmentally regulated B- lymphoid phosphatase that promotes G0/G1 arrest." Blood 94(7): 2403-13.
- Boissier MC et al. Biphasic effect of interferon-gamma in murine collagen-induced arthritis. Eur. J. Immunol., 25: 1184-1190, 1995
- Cuzzocrea S. et al. Reduction in the evolution of murine type II collagen-induced arthritis by treatment with rosiglitazon, a ligand of the peroxisome proliferator-activated receptor $\tilde{\gamma}$. Arthr. And Rheum., 48: 3544-3556, 2003
- Harris ED Jr. Rheumatoid arthritis: pathophysiology and implications for therapy. N. Engl. J. Med., 322: 1277-1289,1990
- Hom JT et al. Interleukin-1 mediated acceleration of type II collagen-induced arthritis: effects of anti-inflammatory or anti-arthritic drugs. Agents Action, 33: 300-309, 1991
- Mori, Y., J. Yin, F. Sato, A. Sterian, L. A. Simms, F. M. Selaru, K. Schulmann, Y. Xu, A. Olaru, S. Wang, E. Deacu, J. M. Abraham, J. Young, B. A. Leggett and S. J. Meltzer (2004). "Identification ofgenes uniquely involved in frequent microsatellite instability colon carcinogenesis by expression profiling combined with epigenetic scanning." Cancer Res. 64(7): 2434-8.
- Motiwala, T., H. Kutay, K. Ghoshal, S. Bai, H. Seimiya, T. Tsuruo, S. Suster, C. Morrison and S. T. Jacob (2004). "Protein tyrosine phosphatase receptor-type O (PTPRO) exhibits characteristics of a candidate tumor suppressor in human lung cancer." Proc. Natl. Acad. Sci. U. S. A. 101(38): 13844-9.
- Niels Peter Hundahl Moller et al. Current Opinion in Drug Discovery & Development 3(5), 527-540 (2000)
- Pixley, F. J., P. S. Lee, J. S. Condeelis and E. R. Stanley (2001). "Protein tyrosine phosphatase phi regulates paxillin tyrosine phosphorylation and mediates colony-stimulating factor 1-induced morphological changes in macrophages."

Mol. Cell. Biol. 21 (5): 1795-809.
- Pixley, F. J., P. S. Lee, M. G. Dominguez, D. B. Einstein and E. R. Stanley (1995). "A heteromorphic protein-tyrosine phosphatase, PTP phi, is regulated by CSF-1 in macrophages." J. Biol. Chem. 270(45): 27339-47.
- S. Wächli et al. : J. Biol. Chem. 2000, 275(13), pp 9792-9796
- Seimiya, H. and T. Tsuruo (1993). "Differential expression of protein tyrosine phosphatase genes duringphorbol ester-induced differentiation of human leukemia U937 cells." Cell Growth Differ. 4(12): 1033-9.
- Stuart JM. et al., Collagen autoimmune arthritis. Annu. Rev. Immunol., 2: 199-218, 1984
- Tomas Mustelin et al, Nature, Vol. 5, page 43, January 2005
- Wharram, B. L., M. Goyal, P. J. Gillespie, J. E. Wiggins, D. B. Kershaw, L. B. Holzman, R. C. Dysko, T. L. Saunders, L. C. Samuelson and R. C. Wiggins (2000). "Altered podocyte structure in GLEPPI (Ptpro)-deficient mice associated with hypertension and low glomerular filtration rate." J. Clin. Invest. 106(10): 1281-90.
- WO 02/102359
- WO 03/037328
- WO 2005/011685
- WO 2005/012280
- WO 2005/09773
- WO03/064376

## Claims

1. Use of a Glepp-1 inhibitor for the manufacture of a medicament for the treatment of an autoimmune and/or an inflammatory disorder wherein the Glepp-1 inhibitor is a carboxylic acid of Formula (I) :

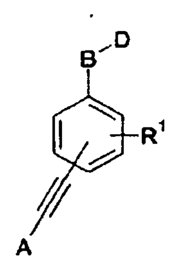

(I)

as well as its geometrical isomers, its optically active forms as enantiomers, diastereomers and its racemate forms, as well as pharmaceutically acceptable salts and pharmaceutically active derivatives thereof, wherein
A is selected from the group consisting of aryl, $C_1$-$C_6$-alkyl aryl;
$R^1$ is selected from the group consisting of H, $C_1$-$C_6$-alkyl, $C_1$-$C_6$-alkoxy, halogen;
B is selected from the group consisting of :

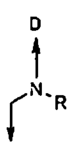

B1

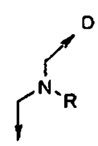

B2

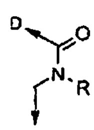

B3

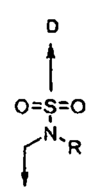

B6

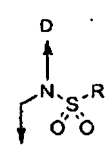

B7

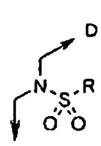

B8

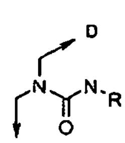

B9

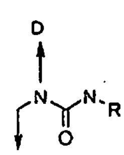

B20

D is either selected from the group consisting of D1, D2, D3 :

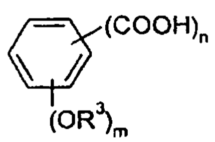

D1

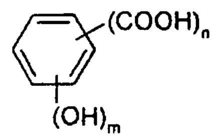

D2

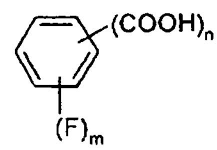

D3

with m being an integer selected from 0, 1 or 2 and n being an integer selected from 1 or 2; and $R^3$ is H or $C_1$-$C_6$ alkyl; or D4

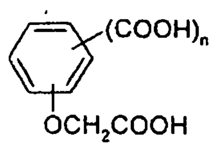

D4

with n being an integer selected from 0 or 1;
R is selected from the group consisting of $C_1$-$C_{12}$-alkyl or $C_1$-$C_8$ alkyl , $C_2$-$C_6$-alkenyl, $C_2$-$C_6$-alkynyl, $C_1$-$C_6$-alkoxy, $C_1$-$C_6$-alkyl amine, $C_1$-$C_6$-alkyl alkoxy, aryl, heteroaryl, saturated or unsaturated $C_3$-$C_8$-cycloalkyl, heterocycloalkyl, $C_1$-$C_6$-alkyl aryl, $C_1$-$C_6$-alkyl heteroaryl, $C_2$-$C_6$-alkenyl aryl, $C_1$-$C_6$-alkenyl heteroaryl, $C_2$-$C_6$-alkynyl aryl, $C_2$-$C_6$-alkynyl heteroaryl, $C_1$-$C_6$-alkyl cycloalkyl, $C_1$-$C_6$-alkyl heterocycloalkyl, $C_2$-$C_6$-alkenyl cycloalkyl, $C_2$-$C_6$-alkenyl heterocycloalkyl, $C_2$-$C_6$-alkynyl cycloalkyl, $C_2$-$C_6$-alkynyl heterocycloalkyl.

**2.** Use according to claim 1, wherein the disorder is selected from the group comprising or consisting of inflammatory bowel diseases, Crohn's disease, ulcerative colitis, collagenous colitis, lymphocytic colitis, diversion colitis irritable bowel syndrome, neuroinflammation including multiple sclerosis; Guillan Barré syndrome, chronic inflammatory polyneuropathy (CIPN), lung diseases (including acute respiratory mistress syndrome; joint and bone diseases including osteoarthritis and rheumatoid arthritis; liver diseases including liver fibrosis, cirrhosis and chronic liver disease; fibrotic diseases including, lupus, glomerulosclerosis, systemic sclerosis skin fibrosis, post-radiation fibrosis and cystic fibrosis; vascular pathologies including atherosclerosis, cardiomyopathy and myocardial infarction; restenosis; and degenerative diseases of the central nervous system including amyotrophic lateral sclerosis; or inflammatory disorders of the skin including scleroderma and psoriasis.

**3.** Use according to claim 1, wherein the Glepp-1 inhibitor is a carboxylic acid of Formula (1a):

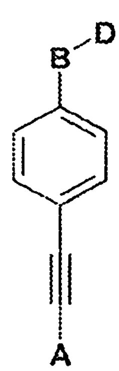

(Ia)

wherein A, B and D are as defined in claim 1.

4. Use according to any of claims 1 to 3, wherein A is $C_4$-$C_6$-akyl aryl, particularly n-butylphenyl.

5. Use according to any of claims 1 to 4, wherein B is B1, B2, B3, B6, B7, B8, B9, or B20, particutarly B1, B2 or B3.

6. Use according to any of claims 1 to 5, wherein R is a $C_4$-$C_6$-alkyl.

7. Use according to any of claims 1 to 6, wherein A is a phenyl group substituted by a $C_1$-$C_4$-alkyl; B is either B1, B2, or B3; R is $C_4$-$C_6$-alkyl, C3-C8-cycloalkyl or $C_1$-$C_6$-alkyl cycloalkyl; and D is selected from:

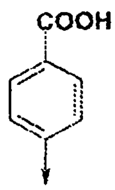
or
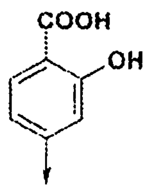
or
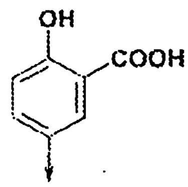
or
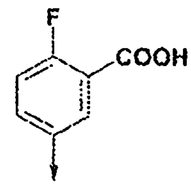

8. Use according to claim 1, wherein the Glepp-1 inhibitor is a carboxylic acid of Formula (1b) or Formula (1c):

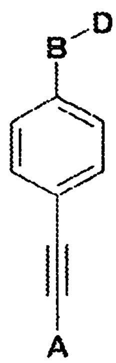

(Ib)

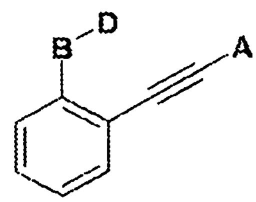

(Ic)

wherein A is a $C_4$-$C_6$-alkyl aryl; B is B1, B3,; and D is as defined in claim 1.

9. Use according to claims to 8, wherein the Glepp-1 Inibitor is detected from the group consisting of
5-[{4-[(4-butylphenyl)ethynyl]benzyl}(hexyl)amino]-2-hydroxybenzoic acid
5-[{4-[(4-butylphenyl)ethynyl)benzyl}(hexyl)amino]-2-fluorobenzoic acid
4-({{4-[(4-butylphenyl)ethynyl]benyl}[2-(4-chlorophenyl)ethyl]amino}-methyl)benzoic acid
5-[{4-[(4-butylphenyl)ethynyl]benzyl}(3-phenylpropyl)amino]-2-hydroxybenzoic acid
5-[{4-[(4-butylphenyl)ethynyl]benzyl}(1-naphthylmethyl)amino]-2-hydroxybenzoic acid
5-((4-tert-butylbenzyl){4-[(4-butylphenyl)ethynyl]benzyl}amino)-2-hydroxybenzoic acid
4-[{4-[(4-butylphenyl)ethynyl]benzyl}(hexyl)amino]-2-hydroxybenzoic acid
2-fluoro-5-{hexyl[4-(phenylethynyl)benzyl]amino}benzoic acid
5-[{4-[(4-butylphenyl)ethynyl]benzyl}(cyclopentylmethyl)amino]-2-fluorobenzoic acid
5-[{4-[(4-butylphenyl)ethynyl]benzyl}(3,3-dimethylbutyl)amino]-2-fluorobenzoic acid
5-[{4-[(4-butylphenyl)ethynyl]benzyl}(ethyl)amino]-2-fluorobenzoic acid
5-(hexyl{4-[(4-propylphenyl)ethynyl]benzyl}amino)-2-hydroxybenzoic acid
5-[{4-[(4-butylphenyl)ethynyl]benzyl}(pentyl)amino]-2-fluorobenzoic acid
5-[{4-[(4-butylphenyl)ethynyl]benzyl}(methyl)amino]-2-fluorobenzoic acid
5-[{4-[(4-butylphenyl)ethynyl]benzyl}(cyclopropylmethyl)amino]-2-fluorobenzoic acid
5-{butyl[4-(phenylethynyl)benzyl]amino}-2-fluorobenzoic acid
2-fluoro-5-[[4-(phenylethynyl)benzyl](propyl)amino]benzoic acid
2-fluoro-5-(hexyl{4-[(4-propylphenyl)ethynyl]benzyl}amino)benzoic acid
5-{{4-[(4-butylphenyl)ethynyl]benzyl}[(2-carboxycyclopropyl)methy]amino}-2-fluorobenzoic acid

5-[{4-[(4-ethylphenyl)ethynyl]benzyl}(hexyl)amino]-2-fluorobenzoic acid
5-[{4-[(4-tert-butylphenyl)ethynyl]benzyl}(hexyl)amino]-2-fluorobenzoic acid
5-[{4-[(4-butylphenyl)ethynyl]benzyl}(isobutyl)amino]-2-fluorobenzoic acid
5-{[{4-[(4-butylphenyl)ethynyl]benzyl}(hexyl)amino]carbonyl}-2-hydroxybenzoic acid
5-{[{4-[(4-butylphenyl)ethynyl]benzoyl}(hexyl)amino]methyl}-2-hydroxybenzoic acid
5- {[{2-[(4-butylphenyl)ethynyl]benzyl}(hexyl)amino]carbonyl}-2-hydroxybenxoic acid
5-{[{4-[(4-butylphenyl)ethynyl]benzyl}(hexyl)amino]carbonyl}-2-ftuorobenzoic acid

**10.** Use according to claim 1, wherein the Glepp-1 Inhibitor is selected from the group consisting of
4-[{4-[(4-butylphenyl)ethynyl]benzyl}(3-cyclcypentylpropanoyl)amino]-2-hydroxy-benzoic acid
5-[{4-[(4-butylphenyl)ethynyl]benxyl}(cyclohexylcarbonyl)amino]-2-hydroxybenzoic acid
5-[{4-[(4-butylphenyl)ethynyl]benzyl}(hexanoyl)amino]-2-hydroxybenzoic acid
5-((4-tert-butylbenzoyl){4-[(4-butylphenyl)ethynyl]benzyl}amino)-2-hydroxybenzoic acid
5-((biphenyl-4-ylcarbonyl){4-[(4-butylphenyl)ethylnyl]benzyl}amino)-2-hydroxybenzoic acid
3-[{4-[(4-butylphenyl)ethynyl]benzyl}(3,3-dimethylbutanoyl)amino]-2-hydroxybenzoic acid
5-((1,3-benzodioxol-5-ylcarbonyl){4-[(4-butylphenyl)ethynyl]benzyl}amino)-2-hydroxybenzoic acid
5-([(benzyloxy)acetyl]{4-[(4-bulylphenyl)ethynyl]benzyl}amino)-2-hydroxybenzoic acid
5-[{4-[(4-butylphenyl)ethynyl]benzyl}(4-hexylbenzoyl)amino]-2-hydroxybenzoic acid
5-((1-benzothien-2-ylcarbonyl){4-[(4-butylphenyl)ethynyl]benzyl}amino)-2-hydroxybenzoic acid
5-{[{4-[(4-butylphenyl)ethynyl]benzyl}(hexanoyl)amino]methyl}-2-hydroxybenzoic acid
(4-{[{4-[(4-butylphenyl)ethynyl]benzyl}(hexanoyl)amino]methyl}phenoxy)acetic acid
4-{[{4-[(4-butylphenyl)ethynyl]benzyl}(3-cyclopentylpropanoyl)amino]methyl}-benzoic acid
5-[{4-[(4-butylphenyl)ethynyl]benzyl}(3-cyclopentylpropanoyl)amino]-2-fluorobenzoic acid
5-[{4-[(4-butylphenyl)ethynyl]benzyl}(3,3-dimethylbutanoyl)amino]-2-fluorobenzoic acid
4-[{4-[(4-butylphenyl)ethynyl]benzyl}(cyclohexylcarbonyl)amino]-2hydroxybenzoic acid
4-[{4-[(4-butylphenyl)ethynyl]benzyl}(hexanoyl)amino]-2-hydroxybenzoic acid
4-[{4-[(4-butylphenyl)ethynyl]benzyl}(3-cyclopentylpropanoyl)amino]-2-fluorobenzoic acid
4-[{4-[(4-tert-butylphenyl)ethynyl]benzyl}(3-cyclopentylpropanoyl)amino]-2-hydroxybenzoic acid
4-((3-cyclopentylpropanoyl){4-[(4-propylphenyl)ethynyl]benzyl}amino)-2-hydroxybenzoic acid
5-{[{4-[(4-butylphenyl)ethynyl]benzyl}(3-cyclopentylpropanoyl)amino]methyl}-2-hydroxybenzoic acid

**11.** Use according to any of the preceding claims whereby the Glepp-1 inhibitor is administered in combination with a co-agent useful in the treatment of an autoimmune and/or an inflammatory disorder.

**12.** A pharmaceutical composition comprising a Glepp-1 inhibitor according to claim 1, a co-agent useful in the treatment of an autoimmune and/or an inflammatory disorder and a pharmaceutically acceptable excipient.

**13.** A pharmaceutical composition according to claim 12, wherein the co-agent is interferon beta.

## Patentansprüche

**1.** Verwendung eines Glepp-1-Inhibitors für die Herstellung eines Medikaments zur Behandlung einer Autoimmun- und/oder einer entzündlichen Erkrankung, wobei der Glepp-1-Inhibitor eine Carbonsäure der Formel (I) ist:

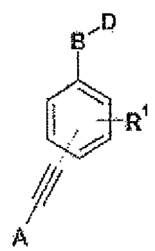

(I)

sowie seine geometrischen Isomere, seine optisch aktiven Formen als Enantiomere, Diastereomere und seine razemischen Formen, sowie pharmazeutisch akzeptable Salze und pharmazeutisch aktive Derivate davon, wobei A ausgewählt ist aus der Gruppe bestehend aus Aryl, $C_1$-$C_6$-Alkylaryl;

$R^1$ ausgewählt ist aus der Gruppe bestehend aus H, $C_1$-$C_6$-Alkyl, $C_1$-$C_6$-Alkoxy, Halogen;
B ausgewählt ist aus der Gruppe bestehend aus:

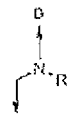
B1
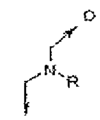
B2
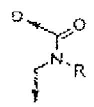
B3
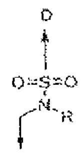
B6
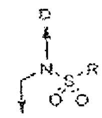
B7

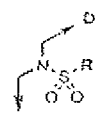
B8
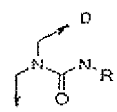
B9
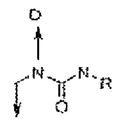
B20

D entweder aus der Gruppe bestehend aus D1, D2, D3 ausgewählt ist:

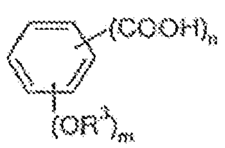
D1
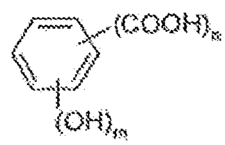
D2
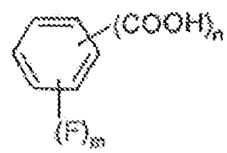
D3

wobei m eine ganze Zahl ausgewählt aus 0, 1 oder 2 ist und n eine ganze Zahl ausgewählt aus 1 oder 2 ist;
und $R^3$ H oder $C_1$-$C_6$-Alkyl ist; oder D4

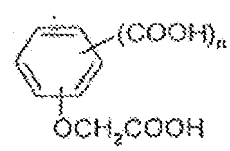
D4

wobei n eine ganze Zahl ausgewählt aus 0 oder 1 ist;
R ausgewählt ist aus der Gruppe bestehend aus $C_1$-$C_{12}$-Alkyl oder $C_1$-$C_5$-Alkyl, $C_2$-$C_6$-Alkenyl, $C_2$-$C_6$-Alkinyl, $C_1$-$C_6$-Alkoxy, $C_1$-$C_6$-Alkylamin, $C_1$-$C_6$-Alkylalkoxy, Aryl, Heteroaryl, gesättigtem oder ungesättigtem $C_3$-$C_8$-Cycloalkyl, Heterocycloalkyl, $C_1$-$C_6$-Alkylaryl, $C_1$-$C_6$-Alkylheteroaryl, $C_2$-$C_6$-Alkenylaryl, $C_2$-$C_6$-Alkenylheteroaryl, $C_2$-$C_6$-Alkinylaryl, $C_2$-$C_6$-Alkinylheteroaryl, $C_1$-$C_6$-Alkylcycloalkyl, $C_1$-$C_6$-Alkylheterocycloalkyl, $C_2$-$C_6$-Alkenylcycloalkyl, $C_2$-$C_6$-Alkenylheterocycloalkyl, $C_2$-$C_6$-Alkinylcycloalkyl, $C_2$-$C_6$-Alkinylheterocycloalkyl.

**2.** Verwendung gemäß Anspruch 1, wobei die Erkrankung ausgewählt ist aus der Gruppe oder bestehend aus entzündlichen Darmerkrankungen, Morbus Crohn, Colitis ulcerosa, kollagener Colitis, lymphozytärer Colitis, Diversionscolitis, Reizdarmsyndrom, Neuroinflammation einschließlich Multipler Sklerose; Guillan-Barré-Syndrom, chronisch entzündlicher Polyneuropathie ("chronic inflammatory polyneuropathy", CIPN), Lungenerkrankungen einschließlich akutem Atemwegssyndrom; Gelenk- und Knochenerkrankungen einschließlich Osteoarthritis und rheumatoider Arthritis; Lebererkrankungen einschließlich Leberfibrose, Zirrhose und chronischer Lebererkrankung; fibrosierenden Erkrankungen einschließlich Lupus, Glomerulosklerose, systemischer Sklerose, Hautfibrose, Fibrose

nach Bestrahlung und zystischer Fibrose; vaskulären Pathologien einschließlich Atherosklerose, Kardiomyopathie und Myokardinfarkt, Restenose; und degenerativen Erkrankungen des zentralen Nervensystems einschließlich amyotropher Lateralsklerose oder entzündlichen Erkrankungen der Haut einschließlich Sklerodermie und Psoriasis.

**3.** Verwendung gemäß Anspruch 1, wobei der Glepp-1-Inhibitor eine Carbonsäure der Formel (Ia) ist:

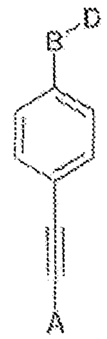

(Ia)

wobei A, B und D wie in Anspruch 1 definiert sind.

**4.** Verwendung gemäß einem der Ansprüche 1 bis 3, wobei A $C_4$-$C_6$-Alkylaryl ist, insbesondere n-Butylphenyl.

**5.** Verwendung gemäß einem der Ansprüche 1 bis 4, wobei B B1, B2, B3, B6, B7, B8, B9 oder B20 ist, insbesondere B1, B2 oder B3.

**6.** Verwendung gemäß einem der Ansprüche 1 bis 5, wobei R ein $C_4$-$C_6$-Alkyl ist.

**7.** Verwendung gemäß einem der Ansprüche 1 bis 6, wobei A eine Phenylgruppe ist, die durch ein $C_1$-$C_4$-Alkyl substituiert ist; B entweder B1, B2 oder B3 ist; R $C_4$-$C_6$-Alkyl, $C_3$-$C_8$-Cycloalkyl oder $C_1$-$C_6$-Alkylcycloalkyl ist; und D ausgewählt ist aus;

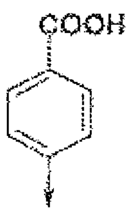 oder 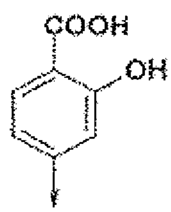 oder 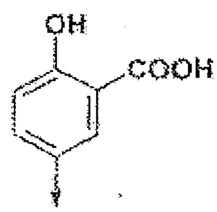 oder 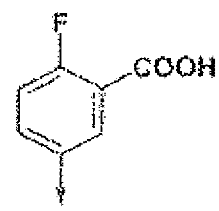

**8.** Verwendung gemäß Anspruch 1, wobei der Glepp-1-Inhibitor eine Carbonsäure der Formel (Ib) oder der Formel (Ic) ist:

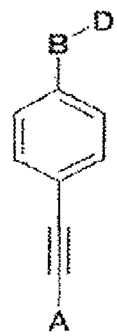

(Ib)

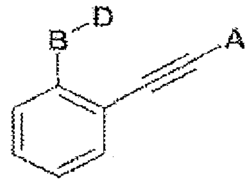

(Ic)

wobei A ein $C_1$-$C_6$-Alkylaryl ist; B B1, B3 ist; und D wie in Anspruch 1 definiert ist.

**9.** Verwendung gemäß Ansprüchen 1 bis 8, wobei der Glepp-1-Inibitor ausgewählt ist aus der Gruppe bestehend aus
5-[{4-[(4-Butylphenyl)ethinyl]benzyl}(hexyl)amino]-2-hydroxybenzoesäure AS606244
5-[{4-[(4-Butylphenyl)ethinyl]benzyl}(hexyl)amino]-2-fluorbenzoesäure
4-({{4-[(4-Butylphenyl)ethinyl]benzyl}[2-(4-chlorphenyl)ethyl]amino}-methyl)benzoesäure
5-[{4-[(4-Butylphenyl)ethinyl]benzyl}(3-phenylpropyl)amino]-2-hydroxybenzoesäure
5-[{4-[(4-Butylphenyl)ethinyl]benzyl}(1-naphthylmethyl)amino]-2-hydroxybenzoesäure
5-((4-tert-Butylbenzyl){4-[(4-butylphenyl)ethinyl]benzyl}amino)-2-hydroxybenzoesäure
4-[{4-[(4-Butylphenyl)ethinyl]benzyl}(hexyl)amino]-2-hydroxybenzoesäure
2-Fluor-5-(hexyl[4-(phenylethinyl)benzyl]amino)benzoesäure
5-[{4-[(4-Butylphenyl)ethinyl]benzyl}(cyclopentylmethyl)amino]-2-fluorbenzoesäure
5-[{4-[(4-Butylphenyl)ethinyl]benzyl}(3,3-dimethylbutyl)amino]-2-fluorbenzoesäure
5-[{4-[(4-Butylphenyl)ethinyl]benzyl}(ethyl)amino]-2-fluorbenzoesäure
5-(Hexyl{4-[(4-propylphenyl)ethinyl]benzyl}amino)-2-hydroxybenzoesäure
5-[{4-[(4-Butylphenyl)ethinyl]benzyl}(pentyl)amino]-2-fluorbenzoesäure
5-[{4-[(4-Butylphenyl)ethinyl]benzyl}(methyl)amino]-2-fluorbenzoesäure
5-[{4-[(4-Butylphenyl)ethinyl]benzyl}(cyclopropylmethyl)amino]-2-fluorbenzoesäure
5-{Butyl[4-(phenylethinyl)benzyl]amino}-2-fluorbenzoesäure
2-Fluor-5-[[4-(phenylethinyl)benzyl](propyl)amino]benzoesäure
2-Fluor-5-(hexyl{4-[(4-propylphenyl)ethinyl]benzyl}amino)benzoesäure
5-{{4-[(4-Butylphenyl)ethinyl]benzyl}[(2-carboxycyclopropyl)methyl]amino}-2-fluorbenzoesäure
5-[{4-[(4-Ethylphenyl)ethinyl]benzyl}(hexyl)amino]-2-fluorbenzoesäure
5-[{4-[(4-tert-Butylphenyl)ethinyl]benzyl}(hexyl)amino]-2-fluorbenzoesäure
5-[{4-[(4-Butylphenyl)ethinyl]benzyl}(isobutyl)amino]-2-fluorbenzoesäure
5-{[{4-[(4-Butylphenyl)ethinyl]benzyl}(hexyl)amino]carbonyl}-2-hydroxybenzoesäure
5-{[{4-[(4-Butylphenyl)ethinyl]benzoyl}(hexyl)amino]methyl}-2-hydroxybenzoesäure
5-{[{2-[(4-Butylphenyl)ethinyl]benzyl}(hexyl)amino]carbonyl}-2-hydroxybenzoesäure AS 606493
5-{[{4-[(4-Butylphenyl)ethinyl]benzyl}(hexyl)amino]carbonyl}-2-fluorbenzoesäure.

**10.** Verwendung gemäß Anspruch 1, wobei der Glepp-1-Inibitor ausgewählt ist aus der Gruppe bestehend aus
4-[{4-[(4-Butylphenyl)ethinyl]benzyl}(3-cyclopentylpropanoyl)amino]-2-hydroxy-benzoesäure
5-[{4-[(4-Butylphenyl)ethinyl]benzyl}(cyclohexylcarbonyl)amino]-2-hydroxybenzoesäure
5-[{4-[(4-Butylphenyl)ethinyl]benzyl}(hexanoyl)amino]-2-hydroxybenzoesäure
5-((4-tert-Butylbenzoyl){4-[(4-butylphenyl)ethinyl]benzyl}amino)-2-hydroxybenzoesäure
5-((Biphenyl-4-ylcarbonyl){4-[(4-butylphenyl)ethinyl]benzyl}amino)-2-hydroxybenzoesäure
5-[{4-[(4-Butylphenyl)ethinyl]benzyl}(3,3-dimethylbutanoyl)amino]-2-hydroxybenzoesäure
5-((1,3-Benzodioxol-5-ylcarbonyl){4-[(4-butylphenyl)ethinyl]benzyl}amino)-2-hydroxybenzoesäure
5-([(Benzyloxy)acetyl]{4-[(4-butylphenyl)ethinyl]benzyl}amino)-2-hydroxybenzoesäure
5-[{4-[(4-Butylphenyl)ethinyl]benzyl}(4-hexylbenzoyl)amino]-2-hydroxybenzoesäure
5-((1-Benzothien-2-ylcarbonyl){4-[{4-butylphenyl}ethinyl]benzyl}amino)-2-hydroxybenzoesäure
5-{[{4-[(4-Butylphenyl)ethinyl]benzyl}(hexanoyl)amino]methyl}-2-hydroxybenzoesäure

4-{[{4-[(4-Butylphenyl)ethinyl]benzyl}(hexanoyl)amino]methyl}phenoxy)essigsäure
4-{[{4-[(4-Butylphenyl)ethinyl]benzyl}(3-cyclopentylpropanoyl)amino]methyl}-benzoesäure
5-[{4-[(4-Butylphenyl)ethinyl]benzyl}(3-cyclopentylpropanoyl)amino]-2-fluorbenzoesäure
5-[{4-[(4-Butylphenyl)ethinyl]benzyl}(3,3-dimethylbutanoyl)amino]-2-fluorbenzoesäure
4-[{4-[(4-Butylphenyl)ethinyl]benzyl}(cyclohexylcarbonyl)amino]-2-hydroxybenzoesäure
4-[{4-[(4-Butylphenyl)ethinyl]benzyl}(hexanoyl)amino]-2-hydroxybenzoesäure
4-[{4-[(4-Butylphenyl)ethinyl]benzyl}(3-cyclopentylpropanoyl)amino]-2-fluorbenzoesäure
4-[{4-[(4-tert-Butylphenyl)ethinyl]benzyl}(3-cyclopentylpropanoyl)amino]-2-hydroxybenzoesäure
4-((3-Cyclopentylpropanoyl){4-[(4-propylphenyl)ethinyl]benzyl}amino)-2-hydroxybenzoesäure
5-{[{4-[(4-butylphenyl)ethinyl]benzyl}(3-cyclopentylpropanoyl)amino]methyl}-2-hydroxybenzoesäure.

**11.** Verwendung gemäß einem der vorhergehenden Ansprüche, wobei der Glepp-1-Inhibitor in Kombination mit einem weiteren Mittel verabreicht wird, das nützlich in der Behandlung einer Autoimmun- und/oder einer entzündlichen Erkrankung ist.

**12.** Pharmazeutische Zusammensetzung umfassend einen Glepp-1-Inhibitor gemäß Anspruch 1, ein weiteres Mittel, das nützlich in der Behandlung einer Autoimmun-und/oder einer entzündlichen Erkrankung ist und einen pharmazeutisch akzeptablen Hilfsstoff.

**13.** Pharmazeutische Zusammensetzung gemäß Anspruch 12, wobei das weitere Mittel Interferon-beta ist.

## Revendications

**1.** Utilisation d'un inhibiteur de la protéine Glepp-1 en vue de la fabrication d'un médicament conçu pour le traitement d'une maladie auto-immune et/ou d'une maladie inflammatoire, lequel inhibiteur de Glepp-1 est un acide carboxylique de formule (I) :

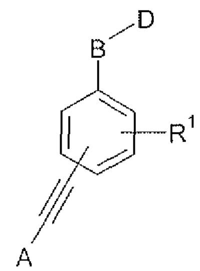

(I)

ou de l'un de ses isomères géométriques, de l'une de ses formes optiquement actives telles que ses énantiomères, diastéréoisoméres et mélanges racémiques, ou de l'un de ses sels pharmacologiquement admissibles ou de ses dérivés pharmacologiquement actifs,
dans laquelle formule :

- A représente une entité choisie parmi les groupes aryle ou (alkyle en $C_{1-6}$)-aryle ;
- $R^1$ représente une entité choisie parmi les atomes d'hydrogène ou d'halogène et les groupes alkyle en $C_{1-6}$ ou alcoxy en $C_{1-6}$ ;
- B représente un fragment choisi parmi ceux de formules suivantes :

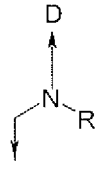

B1

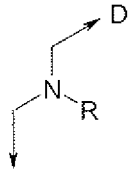

B2

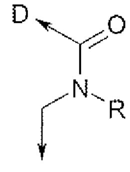

B3

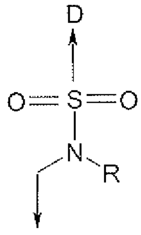

B6

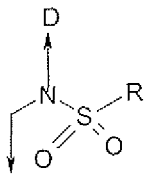 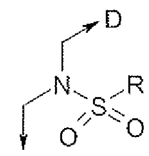 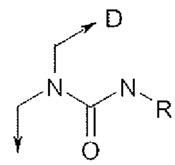 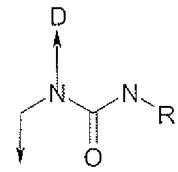

B7 B8 B9 B20

- D représente soit un fragment choisi parmi les fragments D1, D2 et D3 suivants :

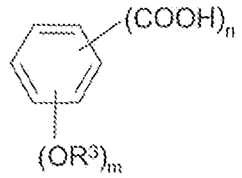 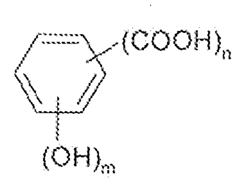 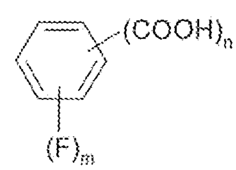

D1 D2 D3

dans lesquels l'indice m est un nombre entier valant 0, 1 ou 2 et l'indice n est un nombre entier valant 1 ou 2, et $R^3$ représente un atome d'hydrogène ou un groupe alkyle en $C_{1-6}$,
soit un fragment D4 :

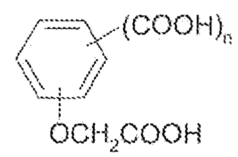

D4

dans lequel l'indice n est un nombre entier valant 0 ou 1 ;
- et R représente une entité choisie parmi les groupes alkyle en $C_{1-12}$ ou alkyle en $C_{1-8}$, alcényle en $C_{2-6}$, alcynyle en $C_{2-6}$, alcoxy en $C_{1-6}$, (alkyle en $C_{1-6}$)-amino, (alkyle en $C_{1-6}$)-alkoxy, aryle, hétéroaryle, cycloalkyle en $C_{3-8}$ saturé ou insaturé, hétérocycloalkyle, (alkyl en $C_{1-6}$)-aryle, (alkyle en $C_{1-6}$)-héteroaryle, (alcényle en $C_{2-6}$)-aryle, (alcényle en $C_{2-6}$)-héteroaryle, (alcynyle en $C_{2-6}$)-aryle, (alcynyle en $C_{2-6}$)-hétéroaryle, (alkyle en $C_{1-6}$)-cycloalkyle, (alkyle en $C_{1-6}$)-hétérocycloalkyle, (alcényle en $C_{2-6}$)-cycloalkyle, (alcényle en $C_{2-6}$)-hetero-cycloalkyle, (alcynyle en $C_{2-6}$)-cycloalkyle, et (alcynyle en $C_{2-6}$)-hétérocycloalkyle.

**2.** Utilisation conforme a la revendication 1, la maladie étant choisie dans l'ensemble formé par les suivantes : maladies inflammatoires intestinales, maladie de Crohn, colite ulcéreuse, colite collagène, colite lymphocytaire, colite de diversion, syndrome de l'intestin irritable, maladies neuro-inflammatoires, y compris la sclérose en plaques, syndrome de Guillain-Barré, polyneuropathie inflammatoire chronique (PNIC), maladies pulmonaires, y compris le syndrome de détresse respiratoire aiguë, maladies des os et des articulations, y compris l'ostéoarthrite et la polyarthrite rhumatoïde, maladies hépatiques, y compris la fibrose hépatique, la cirrhose et l'hépatite chronique, fibroses, y compris le lupus, la glomérulosclérose, la sclérodermie systémique, la fibrose faisant suite à une exposition à des rayonnements et la fibrose kystique, pathologies vasculaires, y compris l'athérosclérose, la cardiomyopathie et l'infarctus du myocarde, resténose, maladies dégénératives du système nerveux central, y compris la sclérose latérale amyotrophique, et maladies inflammatoires de la peau, y compris la sclérodermie et le psoriasis.

**3.** Utilisation conforme à la revendication 1, dans laquelle l'inhibiteur de Glepp-1 est un acide carboxylique de formule (Ia) :

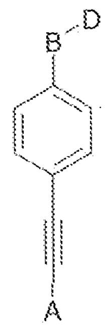

(Ia)

dans laquelle les symboles A, B et D ont les significations indiquées dans la revendication 1.

**4.** Utilisation conforme à l'une des revendication 1 à 3, dans laquelle A représente un groupe (alkyle en $C_{4-6}$)-aryle, en particulier un groupe n-butyl-phényle.

**5.** Utilisation conforme à l'une des revendications 1 à 4, dans laquelle B représente un fragment B1, B2, B3, B6, B7, B8, B9 ou B20, en particulier un fragment B1, B2 ou B3.

**6.** Utilisation conforme à l'une des revendications 1 à 5, dans laquelle R représente un groupe alkyle en $C_{4-6}$.

**7.** Utilisation conforme à l'une des revendications 1 à 3, dans laquelle A représente un groupe phényle à substituant, alkyle en $C_{1-4}$, B représente un fragment B1, B2 ou B3, R représente un groupe alkyle en $C_{4-6}$, cycloalkyle en $C_{3-8}$ ou (alkyle en $C_{1-6}$)-cycloalkyle, et D représente un fragment choisi parmi ceux de formules suivantes :

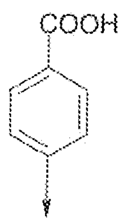 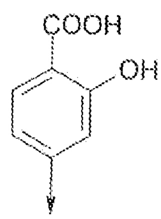 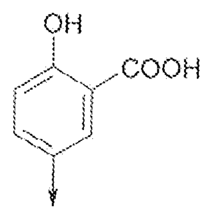 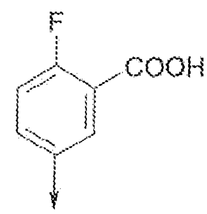

**8.** Utilisation conforme à la revendication 1, dans laquelle l'inhibiteur de Glepp-1 est un acide carboxylique de formule (Ib) ou (Ic) :

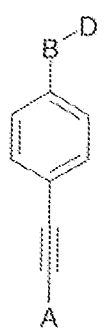 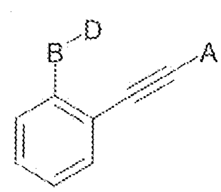

(Ib) (Ic)

dans laquelle A représente un groupe (alkyle en $C_{1-6}$)-aryle, B représente un fragment B1 ou B3, et D a la signification indiquée dans la revendication 1.

**9.** Utilisation conforme à l'une des revendications 1 à 8, pour laquelle l'inhibiteur de Glepp-1 est choisi dans l'ensemble des composés suivants :

acide 5-[{4-[(4-butyl-phényl)éthynyl]benzyl}(hexyl)amino]-2-hydroxy-benzoïque (AS606244)
acide 5-[{4-[(4-butyl-phényl)éthynyl]benzyl}(hexyl)amino]-2-fluoro-benzoïque
acide 4-({{4-[(4-butyl-phényl)éthynyl]benzyl}[2-(4-chloro-phényl)éthyl]-amino}-méthyl)benzoïque
acide 5-[{4-[(4-butyl-phényl)éthynyl]benzyl}(3-phényl-propyl)amino]-2-hydroxy-benzoïque
acide 5-[{4-[(4-butyl-phényl)éthynyl]benzyl}(napht-1-yl-méthyl)amino]-2-hydroxy-benzoïque
acide 5-((4-tertiobutyl-benzyl){4-[(4-butyl-phényl)éthynyl]benzyl}amino]-2-hydroxy-benzoïque
acide 4-[{4-[(4-butyl-phényl)éthynyl]benzyl}(hexyl)amino]-2-hydroxy-benzoïque
acide 2-fluoro-5-{(hexyl)[4-(phényl-éthynyl)benzyl]amino}-benzoïque acide 5-[{4-[(4-butyl-phényl)éthynyl]benzyl}(cyclopentyl-méthyl)amino]-2-fluoro-benzoïque
acide 5-[{4-[(4-butyl-phényl)éthynyl]benzyl}(3,3-diméthyl-butyl)amino]-2-fluoro-benzoïque
acide 5-[{4-[(4-butyl-phényl)éthynyl]benzyl}(éthyl)amino]-2-fluoro-benzoïque
acide 5-[(hexyl){4-[(4-propyl-phényl)éthynyl]benzyl}amino]-2-hydroxy-benzoïque
acide 5-[{4-[(4-butyl-phényl)éthynyl]benzyl}(pentyl)amino]-2-fluoro-benzoïque
acide 5-[{4-[(4-butyl-phényl)éthynyl]benzyl}(methyl)amino]-2-fluoro-benzoïque
acide 5-[{4-[(4-butyl-phényl)ethynyl]benzyl}(cyclopropyl-methyl)amino]-2-fluoro-benzoïque
acide 5-{(butyl)[4-(phényl-ethynyl)benzyl]amino}-2-fluoro-benzoïque acide 2-fluoro-5-{[4-(phényl-éthynyl)benzyl](propyl)amino}-benzoïque acide 2-fluoro-5-[(hexyl){4-[(4-propyl-phényl)éthynyl]benzyl}amino]-benzoïque
acide 5-{{4-[(4-butyl-phényl)éthynyl]benzyl}[(2-carboxy-cyclopropyl)-méthyl]amino}-2-fluoro-benzoïque
acide 5-[{4-[(4-éthyl-phenyl)ethynyl]benzyl}(hexyl)amino]-2-fluoro-benzoïque
acide 5-[{4-[(4-tertiobutyl-phényl)éthynyl]benzyl}(hexyl)amino]-2-fluoro-benzoïque
acide 5-[{4-[(4-butyl-phényl)éthynyl]benzyl}(isobutyl)amino]-2-fluoro-benzoïque
acide 5-{[{4-[(4-butyl-phényl)éthynyl]benzyl}(hexyl)amino]carbonyl}-2-hydroxy-benzoïque
acide 5-{[{4-[(4-butyl-phényl)ethynyl]benzoyl}(hexyl)amino]méthyl}-2-hydroxy-benzoïque
acide 5-{[{2-[(4-butyl-phényl)éthynyl]benzyl}(hexyl)amino]carbonyl}-2-hydroxy-benzoïque (AS606493)
acide 5-{[{4-[(4-butyl-phényl)éthynyl]benzyl}(hexyl)amino]carbonyl}-2-fluoro-benzoïque

**10.** Utilisation conforme à la revendication 1, pour laquelle l'inhibiteur de Glepp-1 est choisi dans l'ensemble des composés suivants :

acide 4-[{4-[(4-butyl-phényl)éthynyl]benzyl}(3-cyclopentyl-propanoyl)-amino]-2-hydroxy-benzoïque
acide 5-[{4-[(4-butyl-phényl)éthynyl]benzyl}(cyclohexyl-carbonyl)amino]-2-hydroxy-benzoïque
acide 5-[{4-[(4-butyl-phenyl)ethynyl]benzyl}(hexanoyl)amino]-2-hydroxy-benzoïque
acide 5-[(4-tertiobutyl-benzoyl){4-[(4-butyl-phényl)éthynyl]benzyl}-amino]-2-hydroxy-benzoïque
acide 5-[{biphényl-4-yl-carbonyl}{4-[(4-butyl-phényl)éthynyl]benzyl}-amino]-2-hydroxy-benzoïque
acide 5-[{4-[(4-butyl-phényl)éthynyl]benzyl}(3,3-diméthyl-butanoyl)-amino]-2-hydroxy-benzoïque
acide 5-[(benzo-1,3-dioxol-5-yl-carbonyl){4-[(4-butyl-phényl)éthynyl]-benzyl}amino]-2-hydroxy-benzoïque
acide 5-[(benzyloxy-acétyl){4-[(4-butyl-phényl)éthynyl]benzyl}amino]-2-hydroxy-benzoïque
acide 5-[{4-[(4-butyl-phenyl)éthynyl]benzyl}(4-hexyl-benzoyl)amino]-2-hydroxy-benzoïque
acide 5-[(benzo-1-thién-2-yl-carbonyl){4-[(4-butyl-phényl)éthynyl]-benzyl}amino]-2-hydroxy-benzoïque
acide 5-{[{4-[(4-butyl-phényl)éthynyl]benzyl}(hexanoyl)amino]méthyl}-2-hydroxy-benzoïque
acide (4-{[{4-[(4-butyl-phényl)éthynyl]benzyl}(hexanoyl)amino]méthyl}-phénoxy)acétique
acide 4-{[{4-[(4-butyl-phényl)éthynyl]benzyl}(3-cyclopentyl-propanoyl)-amino]méthyl}benzoïque
acide 5-[{4-[(4-butyl-phényl)éthynyl]benzyl}(3-cyclopentyl-propanoyl)-amino]-2-fluoro-benzoique
acide 5-[{4-[(4-butyl-phényl)éthynyl]benzyl}(3,3-diméthyl-butanoyl)-amino]-2-fluoro-benzoïque
acide 4-[{4-[(4-butyl-phényl)éthynyl]benzyl}(cyclohexyl-carbonyl)amino]-2-hydroxy-benzoïque
acide 4-[{4-[(4-butyl-phenyl)ethynyl]benzyl}(hexanoyl)amino]-2-hydroxy-benzoïque
acide 4-[{4-[(4-butyl-phényl)éthynyl]benzyl}(3-cyclopentyl-propanoyl)-amino]-2-fluoro-benzoïque
acide 4-[{4-[(4-tertiobutyl-phényl)éthynyl]benzyl}(3-cyclopentyl-propanoyl)amino]-2-hydroxy-benzoïque
acide 4-[(3-cyclopentyl-propanoyl){4-[(4-propyl-phényl)éthynyl]benzyl}-amino]-2-hydroxy-benzoïque
acide 5-{[{4-[(4-butyl-phényl)éthynyl]benzyl}(3-cyclopentyl-propanoyl)-amino]méthyl}-2-hydroxy-benzoïque

**11.** Utilisation conforme à l'une des revendications précédentes, l'inhibiteur de Glepp-1 devant être administré en combinaison avec un co-agent utile dans le traitement d'une maladie auto-immune et/ou d'une maladie inflammatoire.

**12.** Composition pharmaceutique comprenant un inhibiteur de Glepp-1 défini dans la revendication 1, un co-agent utile dans le traitement d'une maladie auto-immune et/ou d'une maladie inflammatoire, et un excipient pharmacologiquement admissible.

**13.** Composition pharmaceutique conforme à la revendication 12, dans laquelle le co-agent est l'interféron bêta.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02080897 A
- EP 727406 A
- WO 2004028251 A
- WO 9955678 A
- WO 0228866 A
- WO 9848802 A
- WO 03068230 A
- WO 0147920 A
- WO 03070711 A
- WO 2004043965 A
- WO 9967230 A
- WO 0145698 A
- US 5540938 A
- WO 9633181 A
- WO 2004080377 A
- WO 03032999 A
- WO 0359871 A
- WO 2005007616 A
- WO 2005097773 A
- WO 02102359 A
- WO 03037328 A
- WO 2005011685 A
- WO 2005012280 A
- WO 200509773 A
- WO 03064376 A


### Non-patent literature cited in the description


- **Niels Peter Hundahl Moller et al.** Protein tyrosine phosphatases (PTPs) as drug targets: Inhibitors of PTP-1B for the treatment of diabetes. *Current Opinion in Drug Discovery & Development,* 2000, vol. 3 (5), 527-540
- **Tomas Mustelin.** *Nature,* January 2005, vol. 5
- Remington's Pharmaceutical Sciences. Marck Publishing Company, 2000
- **S. Wächli et al.** *J. Biol. Chem.,* 2000, vol. 275 (13), 9792-9796
- **Aguiar, R. C. ; Y. Yakushijin ; S. Kharbanda ; S. Tiwari ; G. J. Freeman ; M. A. Shipp.** PTPROt: an alternatively spliced and developmentally regulated B- lymphoid phosphatase that promotes G0/G1 arrest. *Blood,* 1999, vol. 94 (7), 2403-13
- **Boissier MC et al.** Biphasic effect of interferon-gamma in murine collagen-induced arthritis. *Eur. J. Immunol.,* 1995, vol. 25, 1184-1190
- **Cuzzocrea S. et al.** Reduction in the evolution of murine type II collagen-induced arthritis by treatment with rosiglitazon, a ligand of the peroxisome proliferator-activated receptor. *Arthr. And Rheum.,* 2003, vol. 48, 3544-3556
- **Harris ED Jr.** Rheumatoid arthritis: pathophysiology and implications for therapy. *N. Engl. J. Med.,* 1990, vol. 322, 1277-1289
- **Hom JT et al.** Interleukin-1 mediated acceleration of type II collagen-induced arthritis: effects of anti-inflammatory or anti-arthritic drugs. *Agents Action,* 1991, vol. 33, 300-309
- **Mori, Y. ; J. Yin ; F. Sato ; A. Sterian ; L. A. Simms ; F. M. Selaru ; K. Schulmann ; Y. Xu ; A. Olaru ; S. Wang.** Identification ofgenes uniquely involved in frequent microsatellite instability colon carcinogenesis by expression profiling combined with epigenetic scanning. *Cancer Res.,* 2004, vol. 64 (7), 2434-8
- **Motiwala, T. ; H. Kutay ; K. Ghoshal ; S. Bai ; H. Seimiya ; T. Tsuruo ; S. Suster ; C. Morrison ; S. T. Jacob.** Protein tyrosine phosphatase receptor-type O (PTPRO) exhibits characteristics of a candidate tumor suppressor in human lung cancer. *Proc. Natl. Acad. Sci. U. S. A.,* 2004, vol. 101 (38), 13844-9
- **Niels Peter Hundahl Moller et al.** *Current Opinion in Drug Discovery & Development,* 2000, vol. 3 (5), 527-540
- **Pixley, F. J. ; P. S. Lee ; J. S. Condeelis ; E. R. Stanley.** Protein tyrosine phosphatase phi regulates paxillin tyrosine phosphorylation and mediates colony-stimulating factor 1-induced morphological changes in macrophages. *Mol. Cell. Biol.,* 2001, vol. 21 (5), 1795-809
- **Pixley, F. J. ; P. S. Lee ; M. G. Dominguez ; D. B. Einstein ; E. R. Stanley.** A heteromorphic protein-tyrosine phosphatase, PTP phi, is regulated by CSF-1 in macrophages. *J. Biol. Chem.,* 1995, vol. 270 (45), 27339-47

- **Seimiya, H. ; T. Tsuruo.** Differential expression of protein tyrosine phosphatase genes duringphorbol ester-induced differentiation of human leukemia U937 cells. *Cell Growth Differ.,* 1993, vol. 4 (12), 1033-9
- **Stuart JM. et al.** Collagen autoimmune arthritis. *Annu. Rev. Immunol.,* 1984, vol. 2, 199-218
- **Tomas Mustelin et al.** *Nature,* January 2005, vol. 5, 43
- **Wharram, B. L. ; M. Goyal ; P. J. Gillespie ; J. E. Wiggins ; D. B. Kershaw ; L. B. Holzman ; R. C. Dysko ; T. L. Saunders ; L. C. Samuelson ; R. C. Wiggins.** Altered podocyte structure in GLEPPI (Ptpro)-deficient mice associated with hypertension and low glomerular filtration rate. *J. Clin. Invest.,* 2000, vol. 106 (10), 1281-90